# EUROPEAN PATENT APPLICATION

(11) **EP 1 637 064 A1**
(43) Date of publication of application: **22.03.2006**
(21) Application number: 04746736.0
(22) Date of filing: 24.06.2004
(51) Int. Cl.: A61B 1/04

(54) **ENCAPSULATED ENDOSCOPE AND ENCAPSULATED ENDOSCOPE SYSTEM**

(30) Priority: 24.06.2003 JP 2003180138
(71) Applicant: Olympus Corporation, Tokyo 151-0072 (JP)
(72) Inventor: MORI, Takeshi, Machida-shi, Tokyo 1940201 (JP); HONDA, Takemitsu, Hino-shi, Tokyo 1910062 (JP); KIMOTO, Seiichiro, Hachioji-shi, Tokyo 1920045 (JP); SHIGEMORI, Toshiaki, Hachioji-shi, Tokyo 1920023 (JP); SHIMIZU, Hatsuo, Hachioji-shi, Tokyo 1920024 (JP)
(74) Representative: von Hellfeld, Axel
(86) International application number: PCT/JP2004/009267
(87) International publication number: WO 2004/112593

(57) **Abstract**

There is provided a capsule endoscope with less load and low power consumption with respect to signal processing specific to an imaging device. The capsule endoscope includes a storage unit (208) that stores signal processing data necessary for signal processing specific to the imaging device (125) of the capsule endoscope, and a transmitting unit (142) that transmits the signal processing data stored in the storage unit. The signal processing data is a value acquired before shipment of the capsule endoscope. The signal processing data is one of data of a white balance coefficient, data of an image of a chart for color signal processing which is taken by the imaging device, data indicating the address of a defective pixel of the imaging device, and data indicating the offset value of the photoelectric conversion characteristic of a CMOS image sensor. The transmitting unit transmits the signal processing data together with imaged data taken by the imaging device.

## Description

### TECHNICAL FIELD

The present invention relates to a capsule endoscope and a capsule endoscope system.

### BACKGROUND ART

There is known a capsule endoscope (swallowable capsule endoscope for medical use) which is designed as to be swallowable through the mouth of a patient and which can take images of digestive systems, such as a stomach, to gather information on inside the celom of a living body. One capsule endoscope of this type proposed has a capsule which incorporates an illumination unit including an LED or the like, a solid-state imaging device including a CCD, CMOS or the like, and a power supply unit including a battery or the like for driving the illumination unit and solid-state imaging device.

Japanese Patent Application Laid-Open No. 2001-245844 discloses the technology of a capsule endoscope having a white balance capability. The publication describes that the capsule endoscope has an image sensor, a scan circuit thereof, and a signal processing circuit integrated on a same chip and the signal processing circuit has an automatic white balance capability.

If white balancing or the like is performed in the signal processing circuit in the capsule endoscope as done in the capsule endoscope described in Japanese Patent Application Laid-Open No. 2001-245844, however, the circuit scale of the internal circuits increases, thereby increasing the consumed current.

There are two power supply systems proposed for capsule endoscopes: a system which uses a battery and a system which supplies power wirelessly. In either system, the problem occurs if white balancing or the like is performed in the signal processing circuit in the capsule endoscope.

It is an object of the present invention to provide a capsule endoscope that performs signal processing specific to an imaging device with low power consumption without increasing the circuit scale of the internal circuits.

### DISCLOSURE OF THE INVENTION

To solve the problems and to achieve the object, the present invention is characterized by including a storage unit that stores signal processing data necessary for signal processing specific to an imaging device of a capsule endoscope; and a transmitting unit that transmits the signal processing data stored in the storage unit.

In the capsule endoscope, the present invention is characterized in that the signal processing data is a value acquired before shipment of the capsule endoscope in advance.

In the capsule endoscope, the present invention is characterized in that the signal processing data is data of a white balance coefficient to be used when a white balancing process of the imaging device is performed.

In the capsule endoscope, the present invention is characterized in that the signal processing data is data of an image of a chart for color signal processing which is taken by the imaging device.

In the capsule endoscope, the present invention is characterized in that the signal processing data is data indicating an address of a defective pixel of the imaging device.

In the capsule endoscope, the present invention is characterized in that the signal processing data is data indicating an offset value of the photoelectric conversion characteristic of the imaging device.

In the capsule endoscope, the present invention is characterized in that the transmitting unit transmits the signal processing data together with imaged data taken by the imaging device.

In the capsule endoscope, the present invention is characterized in that the transmitting unit transmits the imaged data with at least a part of the signal processing data included in each frame to be a transmission unit at a time of transmitting the imaged data.

In the capsule endoscope, the present invention is characterized in that the signal processing data is added on an end side of the frame.

In the capsule endoscope, the present invention is characterized in that the signal processing data is added to a top end of the frame.

In the capsule endoscope, the present invention is characterized in that the transmitting unit transmits the signal processing data together with an error correction code of the signal processing data.

In the capsule endoscope, the present invention is characterized in that the error correction code is acquired before shipment of the capsule endoscope in advance, and data of the error correction code is stored in the storage unit.

The present invention is a capsule endoscope system that includes a capsule endoscope including a storage unit that stores signal processing data necessary for signal processing specific to an imaging device of the capsule endoscope; and a transmitting unit that transmits the signal processing data stored in the storage unit; and a receiver that receives the signal processing data transmitted from the transmitting unit, characterized in that the capsule endoscope does not perform signal processing specific to the imaging device but the receiver performs signal processing specific to the imaging device based on the received signal processing data.

In the capsule endoscope system, the present invention is characterized in that the signal processing data is a value acquired before shipment of the capsule endoscope in advance.

In the capsule endoscope system, the present invention is characterized in that the signal processing data is data of a white balance coefficient to be used when a white balancing process of the imaging device is performed.

In the capsule endoscope system, the present invention is characterized in that the signal processing data is data of an image of a chart for color signal processing which is taken by the imaging device.

In the capsule endoscope system, the present invention is characterized in that the signal processing data is data indicating an address of a defective pixel of the imaging device.

In the capsule endoscope system, the present invention is characterized in that the signal processing data is data indicating an offset value of the photoelectric conversion characteristic of the imaging device.

In the capsule endoscope system, the present invention is characterized in that the transmitting unit transmits the signal processing data together with imaged data taken by the imaging device.

In the capsule endoscope system, the present invention is characterized in that the transmitting unit transmits the imaged data with at least a part of the signal processing data included in each frame to be a transmission unit at a time of transmitting the imaged data.

In the capsule endoscope system, the present invention is characterized in that the signal processing data is added on an end side of the frame.

In the capsule endoscope system, the present invention is characterized in that the signal processing data is added to a top end of the frame.

In the capsule endoscope system, the present invention is characterized in that the transmitting unit transmits the signal processing data together with an error correction code of the signal processing data.

In the capsule endoscope system, the present invention is
characterized in that the error correction code is acquired before shipment of the capsule endoscope in advance, and data of the error correction code is stored in the storage unit.

### BRIEF DESCRIPTION OF THE DRAWINGS

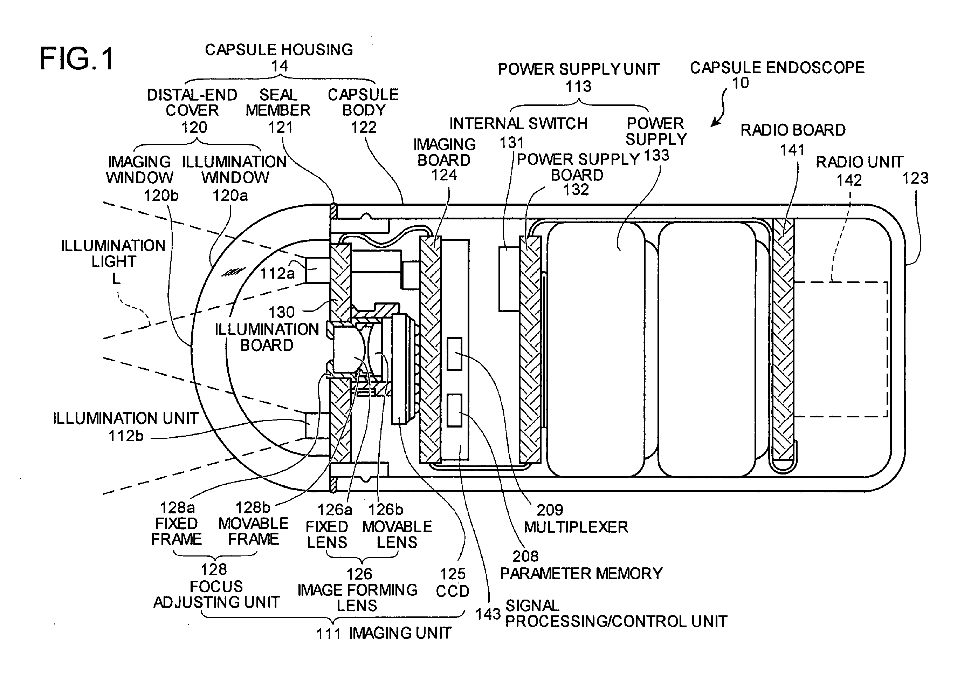

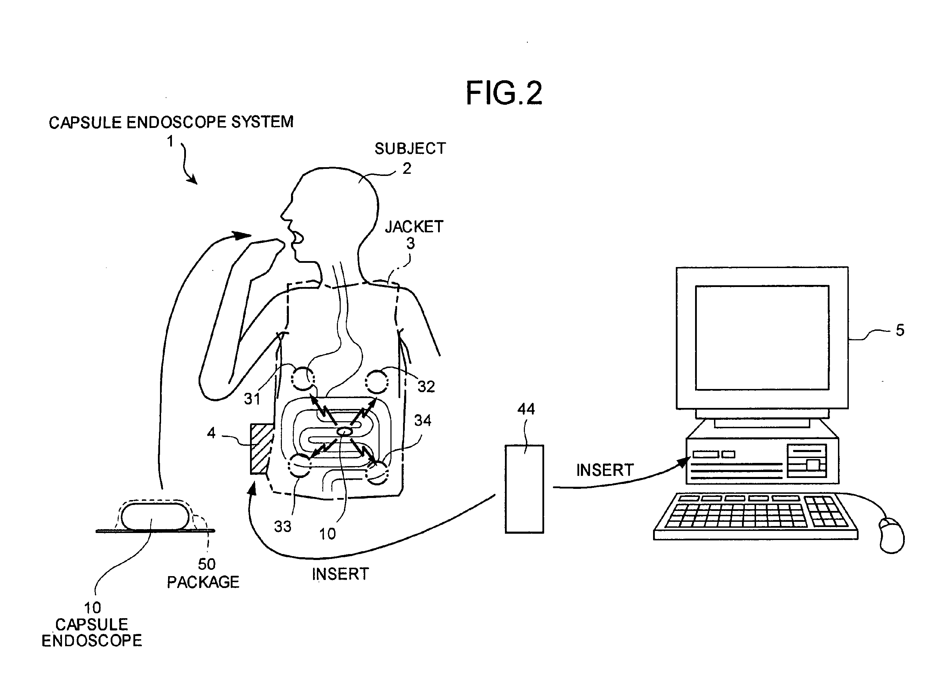

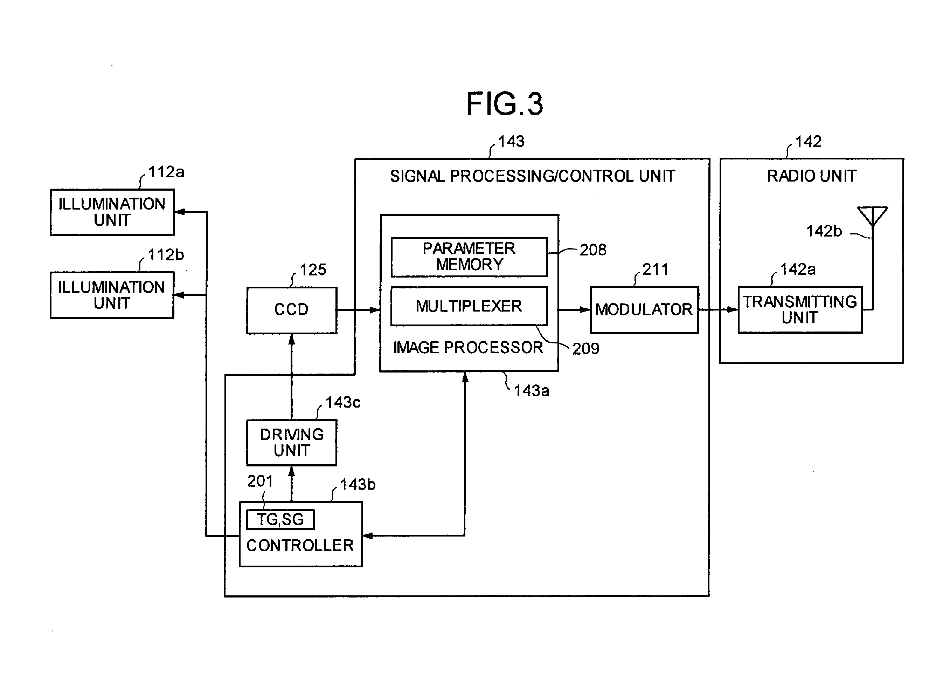

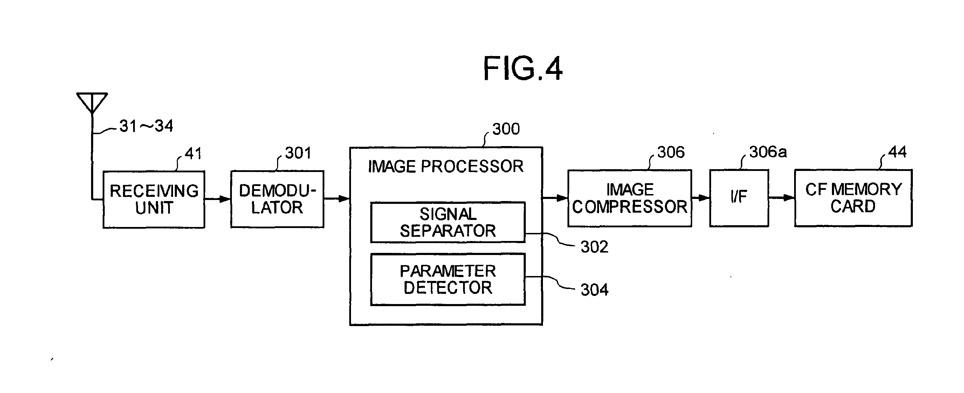

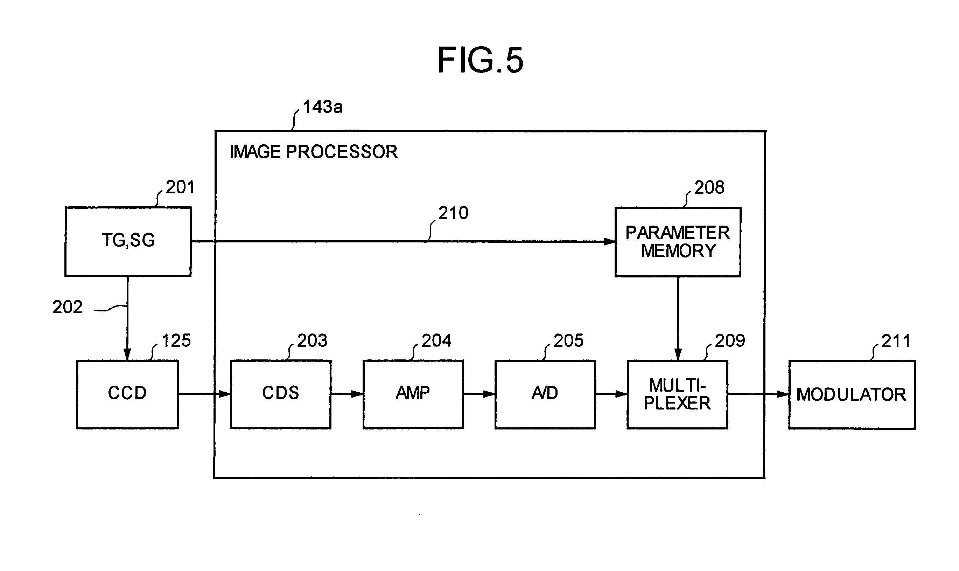

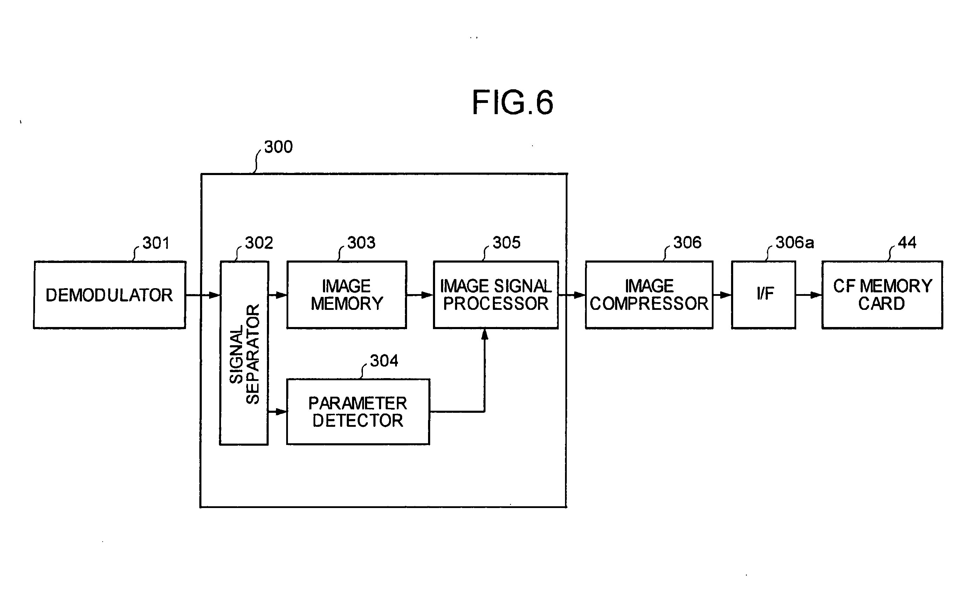

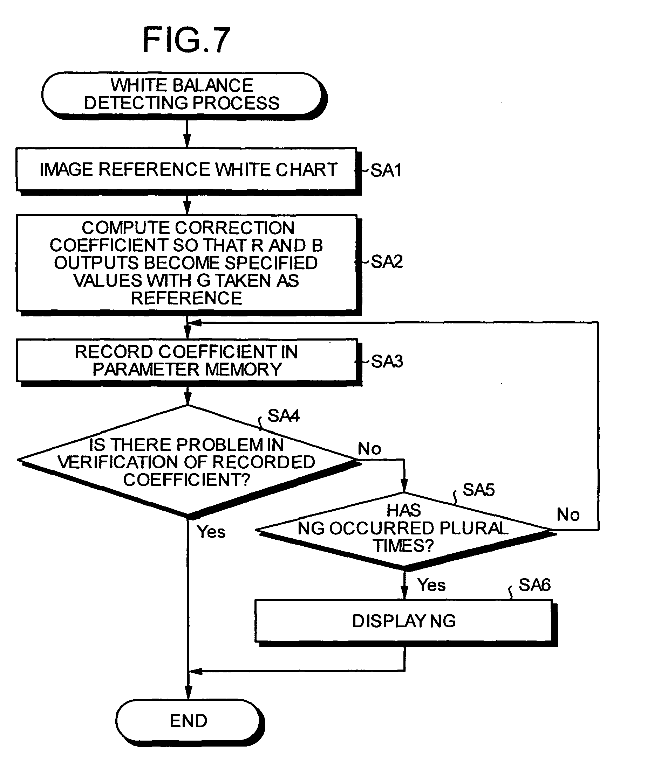

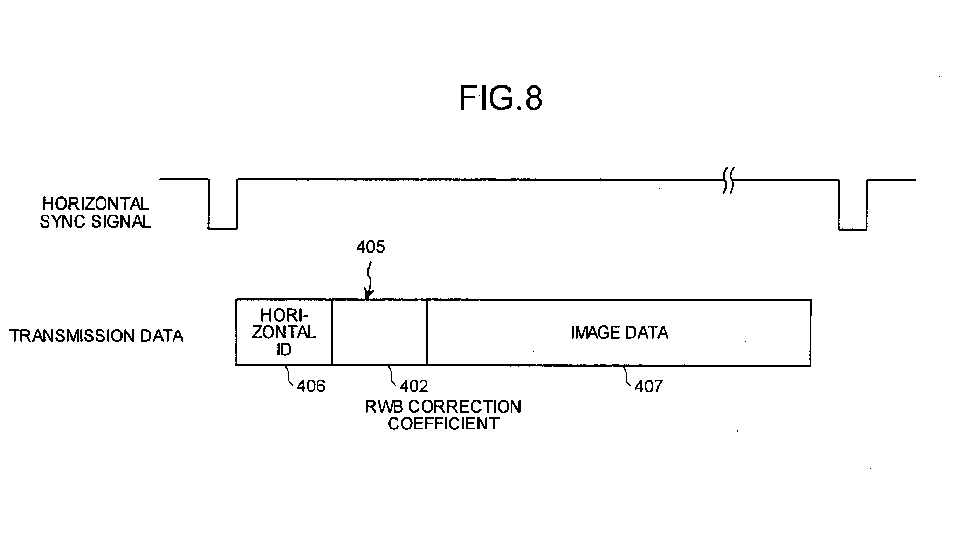

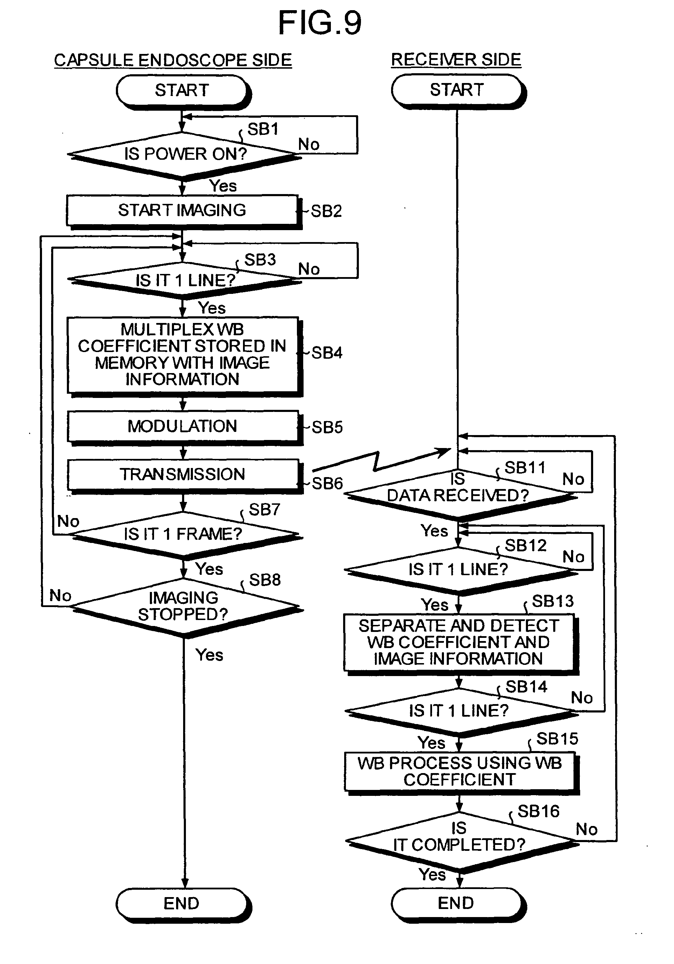

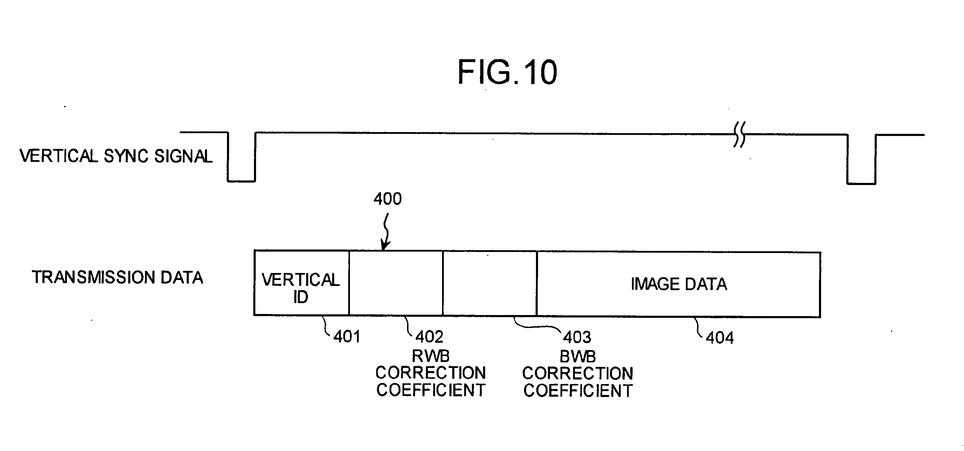

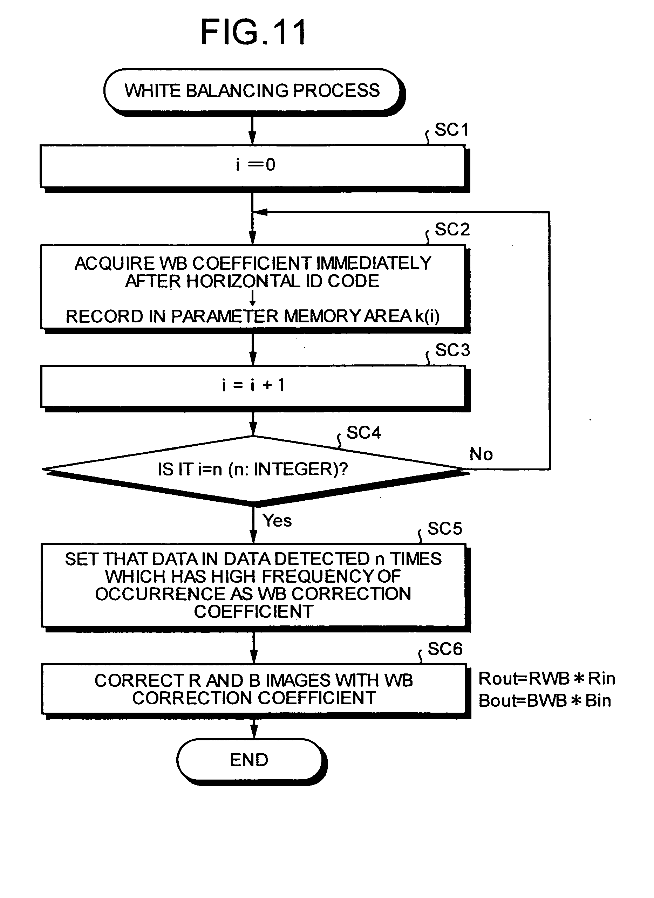

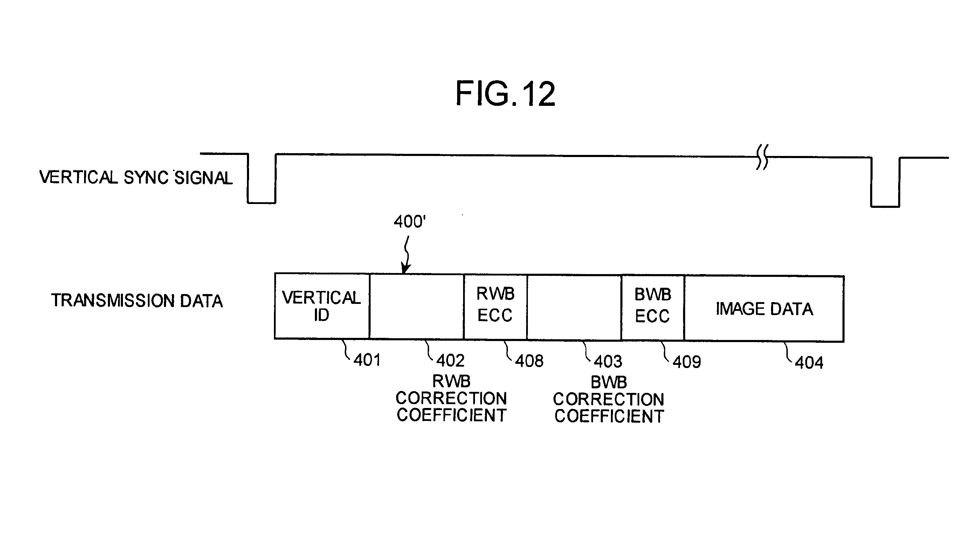

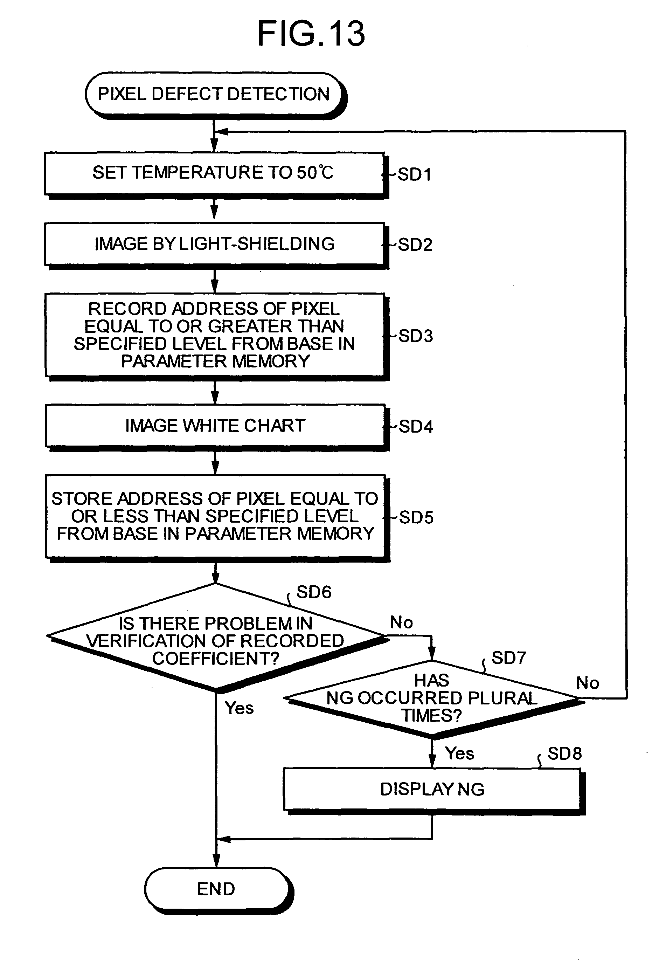

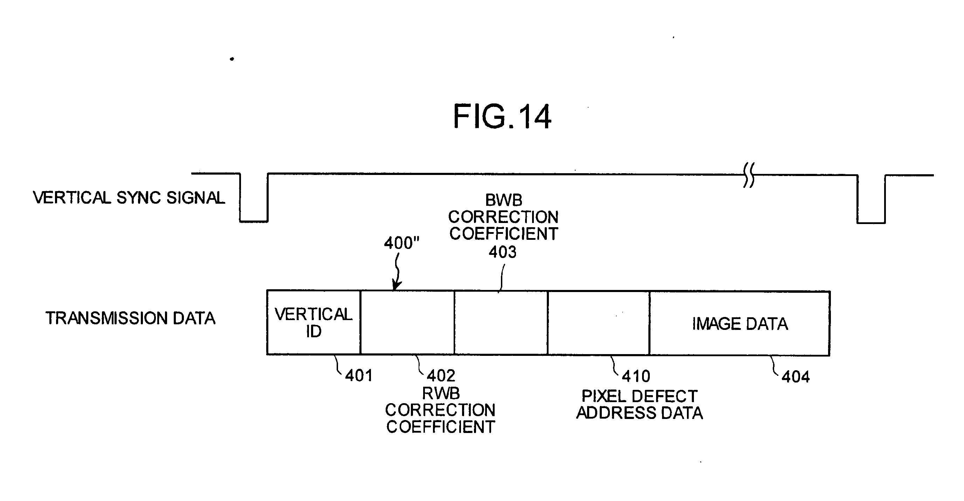

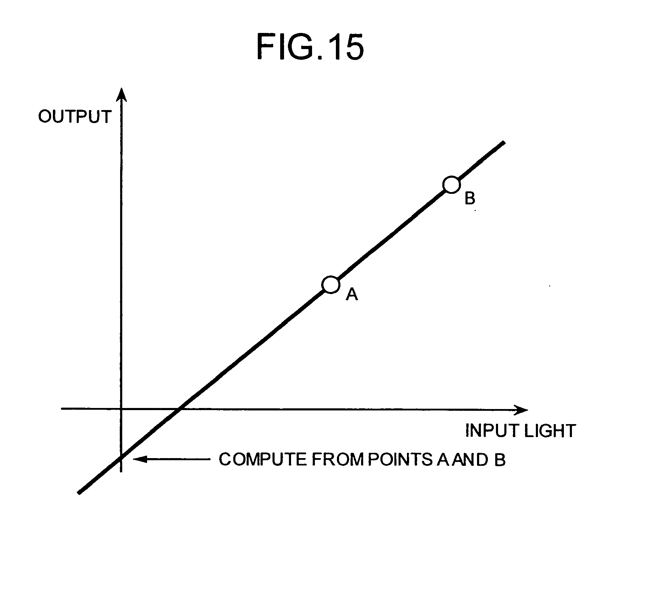

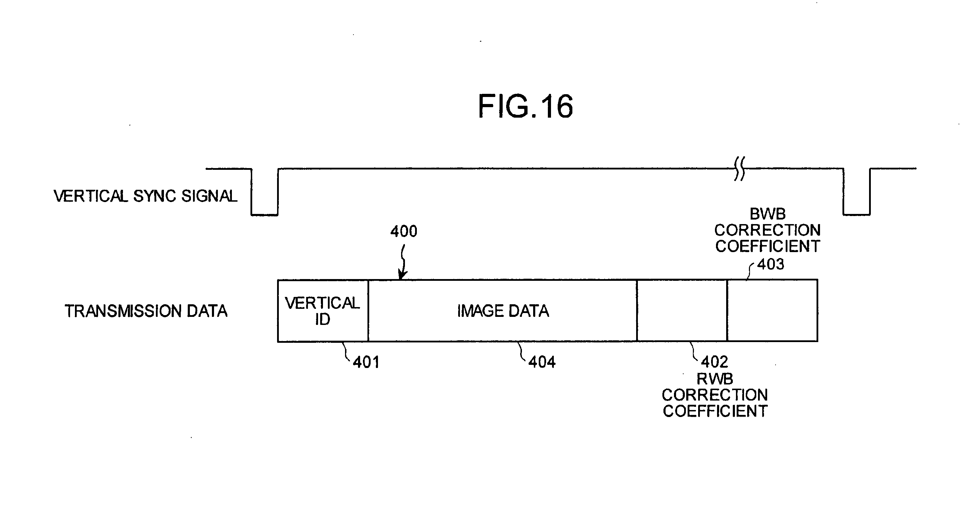

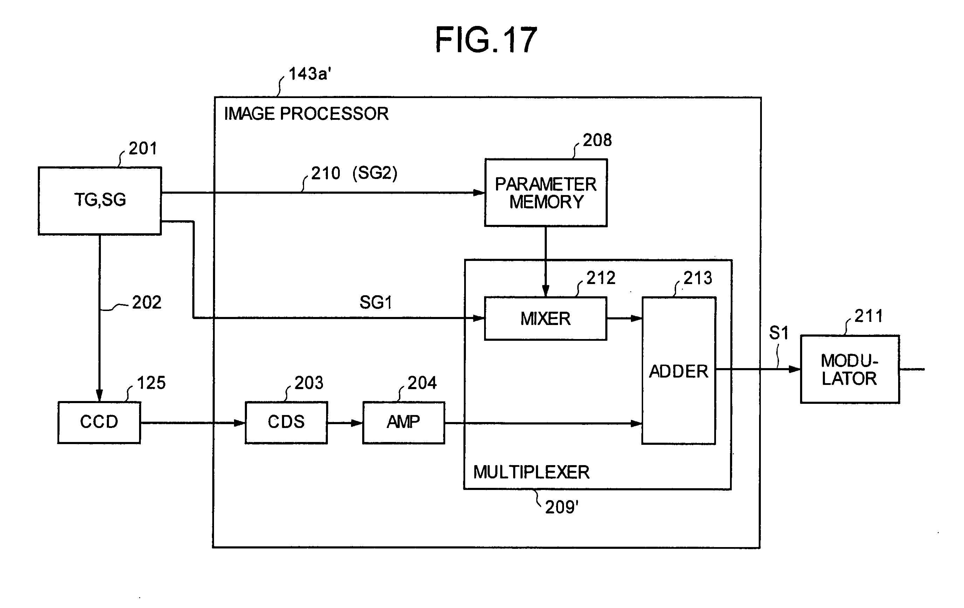

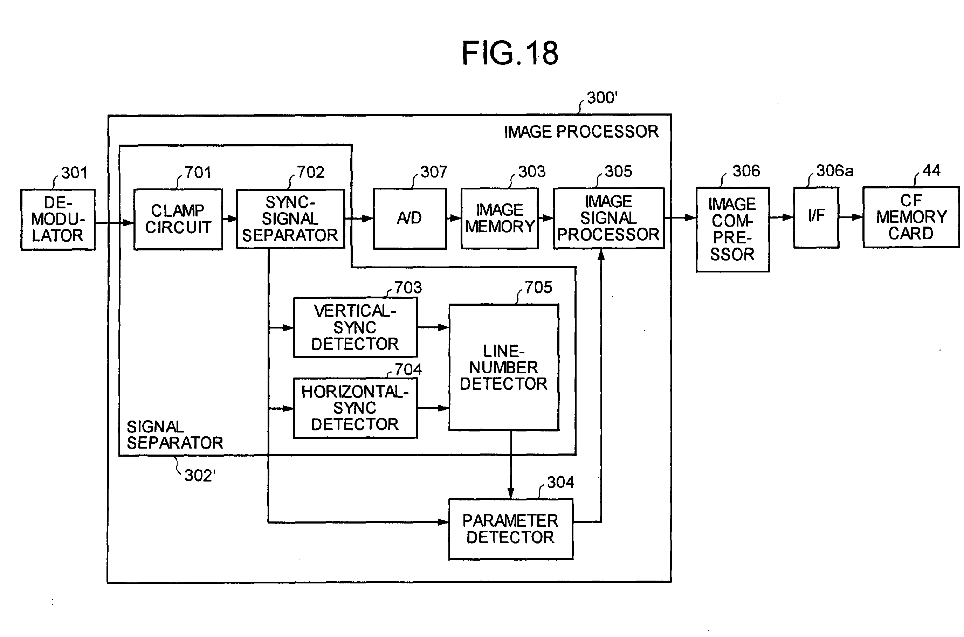

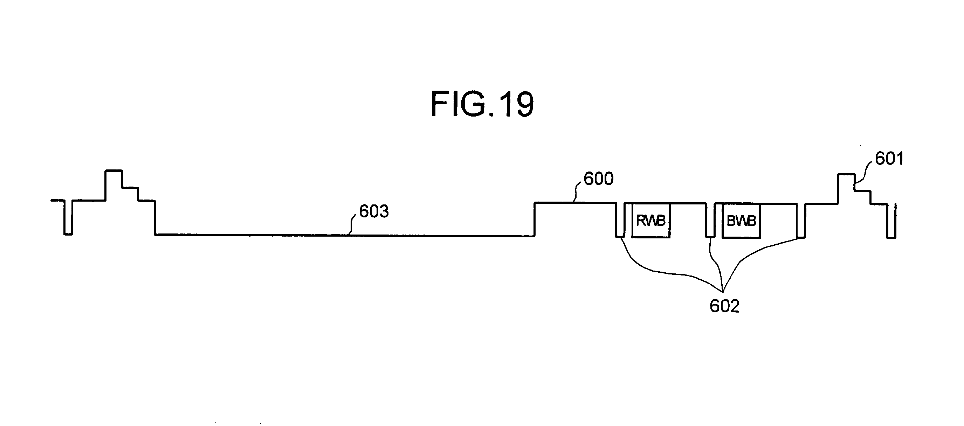

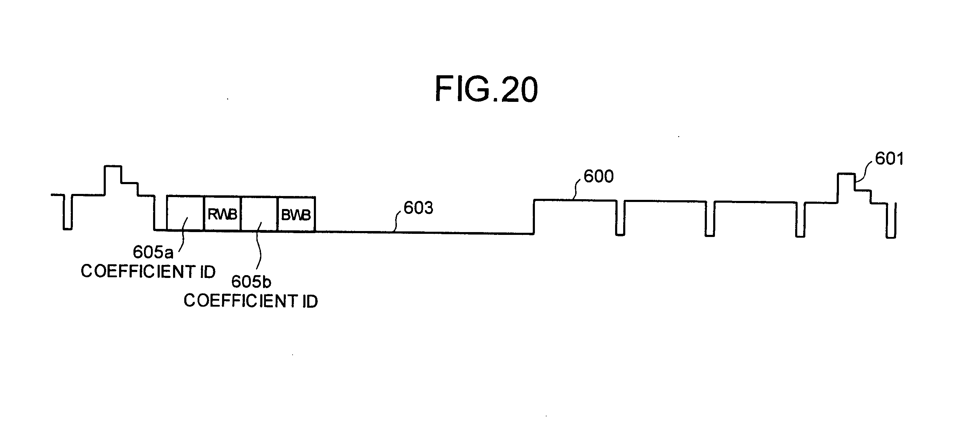

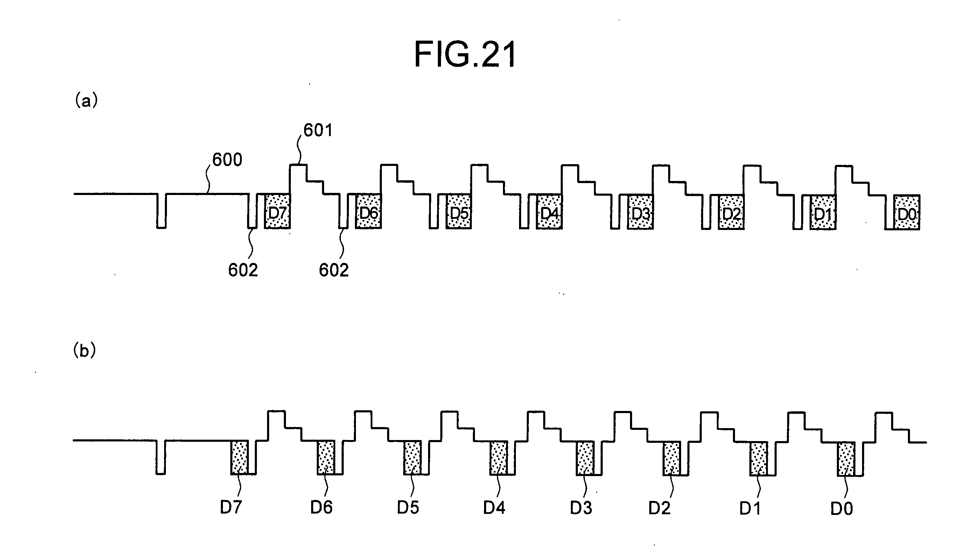

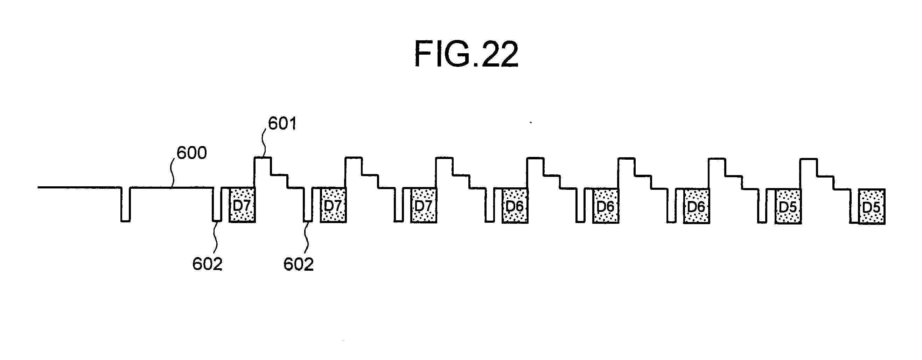

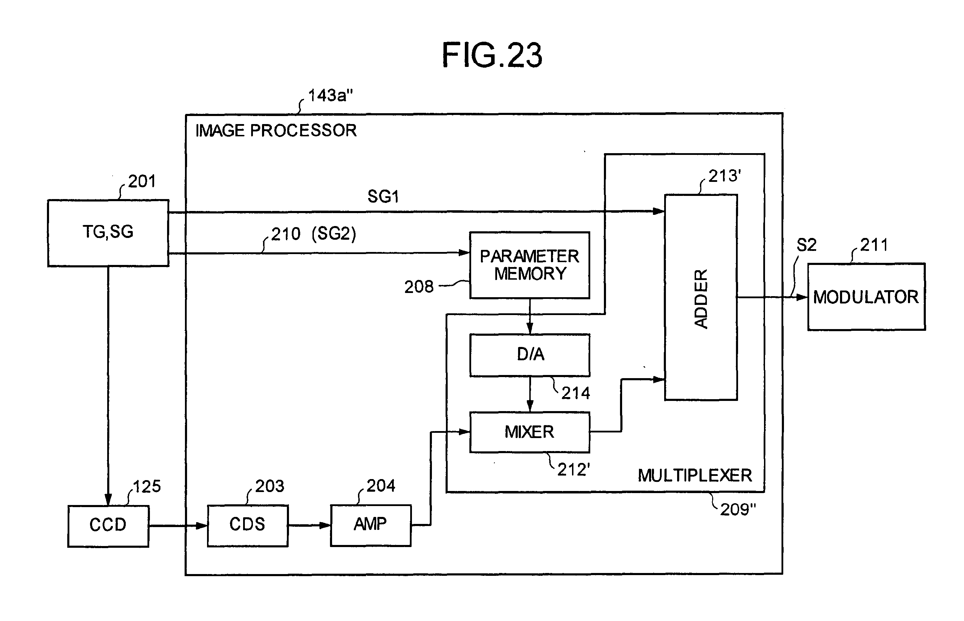

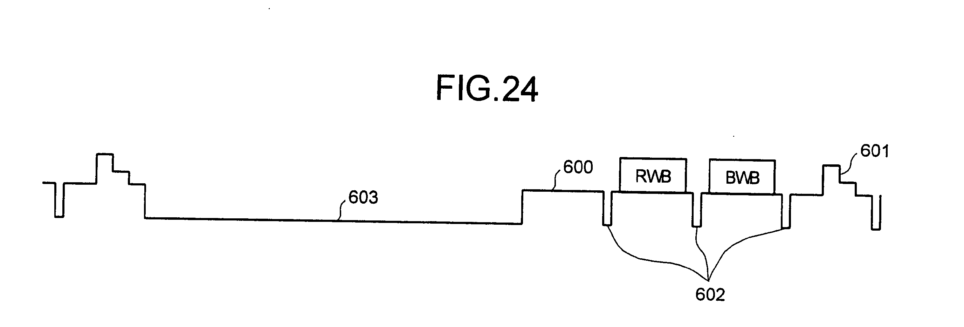

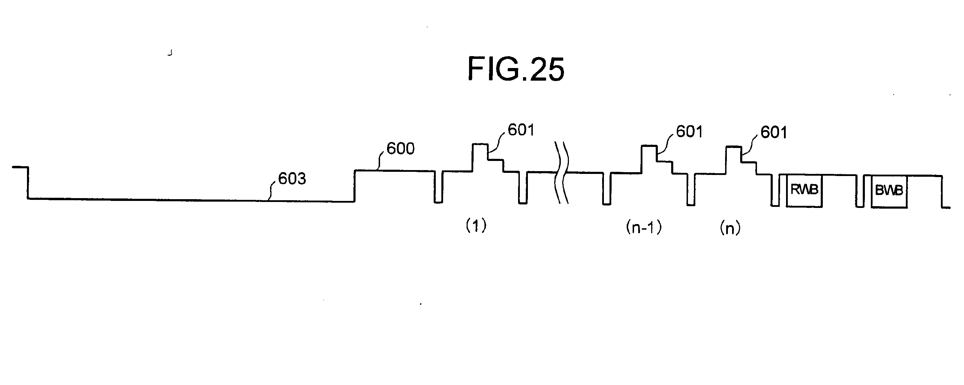

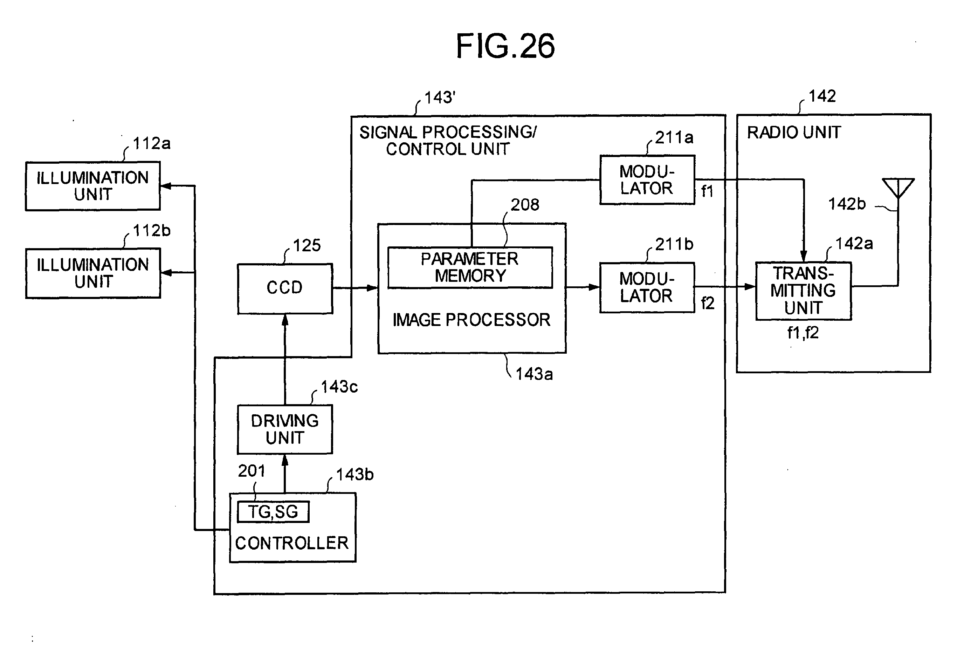

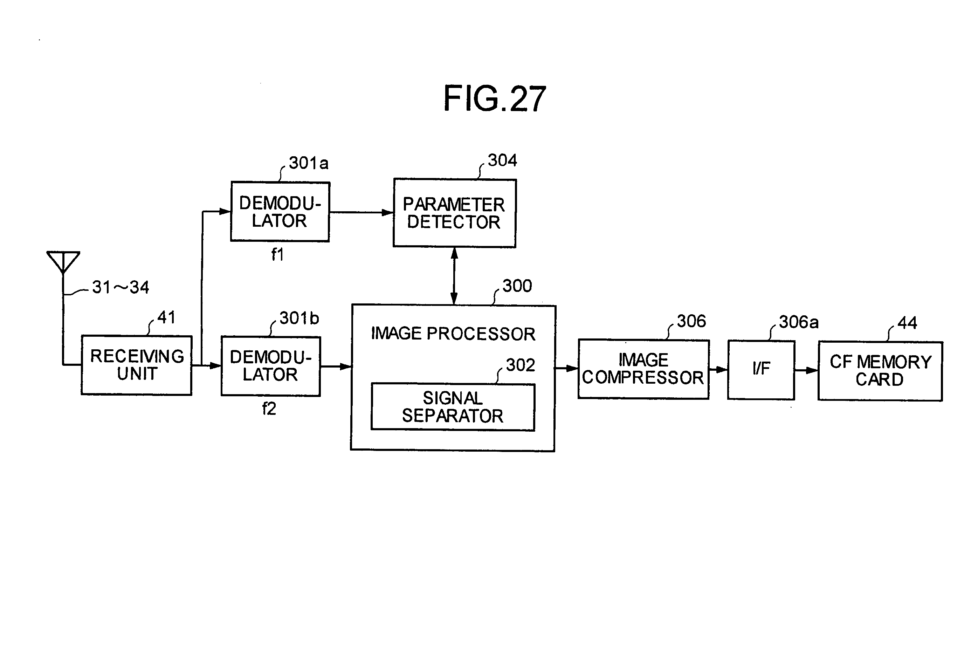

Fig. 1 is a side cross-sectional diagram of a capsule endoscope according to an embodiment of the present embodiment; Fig. 2 is a block diagram of a capsule endoscope system according to the embodiment of the present embodiment; Fig. 3 is a configurational block diagram of the capsule endoscope according to the embodiment of the present embodiment; Fig. 4 is a configurational block diagram of a receiver according to the embodiment of the present invention; Fig. 5 is a configurational block diagram of an image processor of the capsule endoscope according to the embodiment of the present invention; Fig. 6 is a configurational block diagram of the image processor of the receiver according to the embodiment of the present invention; Fig. 7 is a flowchart of procedures of acquiring a white balance coefficient for the capsule endoscope according to the embodiment of the present invention; Fig. 8 is a configurational diagram of a transmission unit of transmission data transmitted from the capsule endoscope according to the embodiment of the present invention; Fig. 9 is a flowchart of operations of the capsule endoscope system according to the embodiment of the present invention; Fig. 10 is a configurational diagram of another transmission unit of transmission data transmitted from the capsule endoscope according to the embodiment of the present invention; Fig. 11 is a flowchart of procedures of a white balancing process executed by the receiver according to the embodiment of the present invention; Fig. 12 is a configurational diagram of still another transmission unit of transmission data transmitted from the capsule endoscope according to the embodiment of the present invention; Fig. 13 is a flowchart of procedures for computing the address of a defective pixel in a capsule endoscope according to another embodiment of the present invention; Fig. 14 depicts still another transmission unit of transmission data including the address of the defective pixel in the capsule endoscope according to the another embodiment; Fig. 15 is an exemplary diagram of a way of acquiring an offset value of photoelectric conversion characteristic of a CMOS image sensor in the capsule endoscope according to the another embodiment; Fig. 16 is an example of adding white balance coefficients at the rear end of image data; Fig. 17 is a configurational block diagram of an image processor of a capsule endoscope according to still another embodiment of the present invention; Fig. 18 is a configurational block diagram of the image processor of a receiver according to the still another embodiment of the present invention; Fig. 19 is a waveform diagram of an output signal from a multiplexer shown in Fig. 17; Fig. 20 is a waveform diagram of another example of the output signal from the multiplexer shown in Fig. 17; Fig. 21 (a) is a waveform diagram of still another example of the output signal form the multiplexer shown in Fig. 17, and Fig. 21 (b) is still another example thereof; Fig. 22 is a waveform diagram of further another example of the output signal from the multiplexer shown in Fig. 17; Fig. 23 is a configurational block diagram of an image processor of a capsule endoscope according to still another example of the present invention; Fig. 24 is an output signal from the multiplexer shown in Fig. 23; Fig. 25 is an example of adding the white balance coefficients at the rear end of a series of video signals; Fig. 26 is a configurational block diagram of a capsule endoscope according to further another embodiment of the present invention; and Fig. 27 is a configurational block diagram of a receiver according to the further another embodiment of the present invention.

### BEST MODE FOR CARRYING OUT THE INVENTION

Exemplary embodiments of the present invention will be explained in detail with reference to the accompanying drawings. However, the invention is not limited by the embodiments.

### (First Embodiment)

The general configuration of a capsule endoscope which is used in one embodiment of the present invention will now be explained with reference to Fig. 1. Fig. 1 is a schematic diagram of the internal configuration of the capsule endoscope according to the present embodiment. As shown in Fig. 1, a capsule endoscope 10 includes an imaging unit 111 that can take internal images of a celom, illumination units 112a and 112b that illuminate the interior of the celom, a power supply unit 113 that supplies power to those units, and a capsule housing 14 which has at least the imaging unit 111, the illumination units 112a and 112b, and the power supply unit 113 disposed inside.

The capsule housing 14 includes a distal-end cover 120 which covers the imaging unit 111 and the illumination units 112a and 112b, and a capsule body 122 which is provided in a water-proof state with respect to the distal-end cover 120 via a seal member 121 and has the imaging unit 111 and the like disposed therein. A rear-end cover 123 may be provided separately from the capsule body 122 as needed. Although the rear-end cover 123 is provided integral with the capsule body 122 and has a flat shape in this embodiment, the shape is not restrictive and may be, for example, a dome shape.

The distal-end cover 120 may be configured to clearly distinguish an illumination window 120a, which transmits illumination light L from the illumination units 112a and 112b, from an imaging window 120b, which performs imaging in the illumination range. In this embodiment, the entire distal-end cover 120 is transparent and the areas of the illumination window 120a and the imaging window 120b partly overlap each other.

The imaging unit 111 is provided on an imaging board 124 and includes a solid-state imaging device 125 formed of, for example, a CCD, which performs imaging in the range that is illuminated with the illumination light L from the illumination units 112a and 112b, and an image forming lens 126 which includes a fixed lens 126a and a movable lens 126b and forms the image of a subject to the solid-state imaging device 125, and executes sharp image forming with a focus adjusting unit 128 including a fixed frame 128a which secures the fixed lens 126a and a movable frame 128b which holds the movable lens 126b.

The imaging unit 111 is not limited to the CCD but an imaging unit, such as a CMOS, may be used.

The illumination units 112a and 112b are provided on an illumination board 130 and each includes, for example, a light-emitting diode (LED). A plurality of illumination units 112a and 112b are laid out around the image forming lens 126 which constitutes the imaging unit 111. In this embodiment, a total of four illumination units are laid out around the image forming lens 126, above, below, right, and left of the image forming lens 126 respectively as one example.

The illumination units 112a and 112b are not limited to the LED but other illumination units may be used as well.

The power supply unit 113 is provided on a power supply board 132 provided with an internal switch 131, and uses, for example, a button type battery as a power supply 133. While a silver oxide cell, for example, is used as the battery in the power supply 133, it is not restrictive. For example, a chargeable battery, a dynamo type battery or the like may be used.

The internal switch 131 is provided to prevent unnecessary current from flowing from the power supply 133 before the capsule endoscope is used.

In this embodiment, a radio unit 142 for radio communication with outside is provided on a radio board 141 and communication with outside is carried out via the radio unit 142 as needed. The radio unit 142 has a transmitting unit 142a that amplifies a signal modulated by a modulator 211, and an antenna 142b, as shown in Figs. 1 and 3.

A signal processing/control unit 143 that processes or controls the above individual units is provided on the imaging board 124 and executes various processing in the capsule endoscope 10. The signal processing/control unit 143 has an image processor 143a, a controller 143b, a driving unit 143c, and the modulator 211.

The image processor 143a has an image signal processing function of generating image data or the like consisting of, for example, correlation double sampling (generally including CDS), and a power supply controlling function of controlling power supply according to the ON/OFF state of the internal switch 131. The image processor 143a also has a parameter memory 208 which stores a parameter, such as a line frame, and a parameter, such as a white balance coefficient, and a multiplexer 209 that multiplexes the white balance coefficient and a video signal.

The controller 143b has a timing generator/sync generator 201 that generates various timing signals or a sync signal. The controller 143b controls the image processor 143a, the driving unit 143c, and the illumination units 112a and 112b based on the timing signals or the sync signal generated by the timing generator/sync generator 201. The illumination units 112a and 112b emit light at given timings in response to the timing signals or the sync signal from the controller 143b.

The driving unit 143c drives the CCD 125 based on the timing signals or the sync signal from the controller 143b.

The controller 143b performs control in such a way that the timing at which the CCD 125 is driven is synchronous with the timing at which the illumination units 112a and 112b emit light, and controls the number of shots taken by the CCD 125.

The modulator 211 has a modulation function of performing conversion to, for example, a PSK, MSK, GMSK, QMSK, ASK, AM, or FM system, and outputs a modulated signal to the transmitting unit 142a.

A capsule endoscope system according to the present embodiment is explained with reference to Fig. 2. Fig. 2 is a schematic diagram of a capsule endoscope system according to the present embodiment. At the time of performing examination using the capsule endoscope 10, the capsule endoscope system 1 as shown in Fig. 2 is used.

A capsule endoscope system 1 according to the present embodiment includes the capsule endoscope 10 and its package 50, a jacket 3 which a patient or a subject 2 wears, a receiver 4 attachable to and detachable from the jacket 3, and a computer 5 as shown in Fig. 2.

The jacket 3 is provided with antennas 31, 32, 33, and 34 that catch radio waves sent from the antenna 142b of the capsule endoscope 10 so as to ensure communication between the capsule endoscope 10 and the receiver 4 via the antennas 31, 32, 33, and 34. The number of antennas is not particularly limited to four but has only to be plural, so that radio waves according to positions of the capsule endoscope 10 moved can be received properly. The position of the capsule endoscope 10 in a celom can be detected according to the reception intensities of the individual antennas 31, 32, 33, and 34.

As shown in Fig. 4, the receiver 4 has a receiving unit 41, a demodulator 301, an image processor 300, an image compressor 306, and a card interface 306a.

The receiving unit 41 amplifies radio wave signals caught by the antennas 31 to 34, and outputs the signals to the demodulator 301.

The demodulator 301 demodulates the output of the receiving unit 41.

The image processor 300 includes a signal separator 302 that performs signal separation on the signals demodulated by the demodulator 301, and a parameter detector 304 that detects a parameter such as a white balance coefficient based on the result of signal separation. The image processor 300 performs white balancing on image data using the detected white balance coefficient.

The image compressor 306 compresses the image data undergone white balancing in the image processor 300.

The card interface 306a has a function of interfacing the input and output of image data between a CF memory card 44 as a large-capacity memory and the image compressor 306.

The CF memory card 44 is detachably mounted on the receiver 4 and stores image data compressed by the image compressor 306.

The receiver 4 is provided with a display unit (not shown) that displays information necessary for observation (examination) and an input unit (not shown) that inputs information necessary for observation (examination).

As shown in Fig. 2, the computer 5 performs reading/writing of the CF memory card 44. The computer 5 has a processing function for a doctor or a nurse (examiner) to perform diagnosis based on images of organs or the like in a patient's body which is imaged by the capsule endoscope 10.

With reference to Fig. 2, the schematic operation of the system will be explained. First, the capsule endoscope 10 is removed from the package 50 before starting examination as shown in Fig. 2. This turns the internal switch 131 in the capsule endoscope 10 ON.

Then, the subject 2 swallows the capsule endoscope 10 with the internal switch 131 turned ON. Accordingly, the capsule endoscope 10 passes through the esophagus, moves inside the celom by peristalsis of the digestive tracts and takes images inside the celom one after another. The radio waves of the taken images are output via the radio unit 142 as needed or at any time for the imaging results, and are caught by the antennas 31, 32, 33, and 34 of the jacket 3. The signals of the caught radio waves are relayed to the receiver 4 from the antenna 31, 32, 33 or 34. At this time, the intensities of the received radio waves differ among the antennas 31, 32, 33, and 34 according to the position of the capsule endoscope 10.

In the receiver 4, white balancing is performed on taken image data which is received piece after piece, and the image data undergone white balancing is stored in the CF memory card 44. Data reception by the receiver 4 is not synchronous with the initiation of imaging by the capsule endoscope 10, and the start of reception and the end of reception are controlled by the manipulation of the input unit of the receiver 4.

When observation (examination) of the subject 2 by the capsule endoscope 10 is finished, the CF memory card 44 where the taken image data are stored is removed from the receiver 4 and is loaded into the memory card slot of the computer 5. The computer 5 reads the taken image data from the CF memory card 44 and stores the image data patient by patient.

With reference to Fig. 5, the image processor 143a of the capsule endoscope 10 will be explained. The image processor 143a shown in Fig. 5 converts analog image data output from the CCD 125 to a digital signal (digital transfer) and sends the digital signal to the modulator 211.

The image processor 143a has a CDS (Correlated Double Sampling) unit 203, an AMP unit 204, an A/D unit 205, the parameter memory 208, and the multiplexer 209.

The timing generator/sync generator 201 provides the CCD 125, at a given timing, with a pulse signal 202 for driving the CCD 125. The pulse (TG) signal 202 is a reference signal to the timing of the imaging system like the CCD 125.

According to the pulse signal 202, charges are read from the CCD 125 after signal conversion. The signals read from the CCD 125 are subjected to noise cancellation by correlated double sampling in the CDS unit 203, thereby generating image data. The image data is amplified by the AMP unit 204, is then subjected to AD conversion in the A/D unit 205, and is then sent to the multiplexer 209.

A white balance coefficient for correcting the white balance is stored in the parameter memory 208. Each capsule endoscope 10 is tested in the fabrication process to acquire a white balance coefficient unique to that capsule endoscope 10. (Acquisition method for the white balance coefficient will be explained later.) The white balance coefficient is written in the parameter memory 208 of each capsule endoscope 10, which is shipped with the unique white balance coefficient stored in the parameter memory 208 of the capsule endoscope 10.

In response to a timing signal 210 output from the timing generator/sync generator 201, the white balance coefficient is read out from the parameter memory 208. The timing (SG) signal 210 is a reference signal to the timing of the display system that constructs an image.

The read out white balance coefficient is superimposed (multiplexed) with the image signal output from the A/D unit 205 by the multiplexer 209, and is then modulated by the modulator 211. As shown in Fig. 3, the modulated signal output from the modulator 211 is sent outside the capsule endoscope 10 via the radio unit 142.

Fig. 6 depicts the configuration of the image processor 300 of the receiver 4 for digital transmission. The image processor 300 has the signal separator 302, an image memory 303, the parameter detector 304, and an image signal processor 305.

Radio waves sent from the radio unit 142 of the capsule endoscope 10 are caught by the antennas 31 to 34. The radio signals are amplified by the receiving unit 41 and then demodulated by the demodulator 301. The signals demodulated by the demodulator 301 are subjected to signal separation in the signal separator 302. Image data is stored in the image memory 303 and the white balance coefficient is detected by the parameter detector 304.

The image signal processor 305 corrects the image data stored in the image memory 303 based on the parameter (white balance coefficient) detected by the parameter detector 304. That is, the image signal processor 305 takes the white balance of the image data based on the white balance coefficient detected by the parameter detector 304.

As apparent from the above, the parameter detected by the parameter detector 304 is a parameter stored in the parameter memory 208 and multiplexed with image data in the multiplexer 209.

The image signal processor 305 performs processing, such as contour enhancement, LPF, and gamma correction, in addition to the image processing for the white balance. The processing, such as contour enhancement, LPF, and gamma correction, unlike the white balancing process, are commonly executed in all the capsule endoscopes 10. Therefore, the parameter for the common processing need not be held in the parameter memory 208 of each capsule endoscope 10, but has only to be stored in the image signal processor 305 as common data to all the capsule endoscopes 10.

The image data corrected by the image signal processor 305 is compressed by the image compressor 306 and is then stored in the CF memory card 44.

Fig. 7 depicts procedures of acquiring the white balance coefficient for each capsule endoscope 10 in the fabrication process.

As shown at step SA1, each capsule endoscope 10 images a white chart to be reference. Next, as shown at step SA2, the correction coefficient (white balance coefficient) is computed in such a way that R (Red) and B (Blue) outputs become specified values with G (Green) taken as a reference. Then, as shown at step SA3, the computed correction coefficient for R and B is recorded in the parameter memory 208.

As shown at step SA4, the correction coefficient recorded in the parameter memory 208 is verified. The verification is to read the correction coefficient from the parameter memory 208 and check if the read correction coefficient matches with the correction coefficient computed at step SA2.

If the verification result shows no problem (if both correction coefficients are identical), detection of the white balance coefficient is finished.

If the verification result shows some problem, it is determined whether the case with the problem (NG) has occurred a predetermined number of times (step SA5). As the case has not occurred a predetermined number of times (NO at SA5), the flow returns to step SA3.

When the occurrence of the case reaches a predetermined number of times at step SA5 (YES at SA5), the presence of an abnormality in the capsule endoscope 10 (particularly in the parameter memory 208) is displayed (step SA6). The capsule endoscope 10 determined as abnormal will not be shipped as it is.

Fig. 8 is a configurational diagram of data format of transmission data (frame) which is the transmission unit when data is transmitted from the capsule endoscope 10 in digital transmission. The transmission unit 405 is composed of data corresponding to one line of the CCD 125.

As shown in Figs. 8 and 5, when a horizontal sync signal (timing data) 210 which is generated by the timing generator/sync generator 201 is input to the parameter memory 208, horizontal identification (ID) data 406 indicating the beginning of one line of data of the CCD 125 and a parameter 402 or 403 of the white balance coefficient are read into the multiplexer 209 in that order from the parameter memory 208 in response to the input horizontal sync signal 210.

When receiving the horizontal ID data 406, the multiplexer 209 starts constructing a new transmission unit 405, has the horizontal ID data 406 and the white balance coefficient 402 for R as the components of the new transmission unit 405 in that order, and adds image data 407, input from the A/D unit 205 before the inputting of the horizontal ID data 406, as a component of the transmission unit 405 after the last component of the transmission unit 405.

The "image data 407, input from the A/D unit 205 before the inputting of the horizontal ID data 406" corresponds to one line of image data of the CCD 125 to whose horizontal shift register (not shown) charges of the CCD 125 are transferred in a horizontal retrace line period. In the transmission unit 405, the white balance coefficient 402 is added to a place corresponding to the time other than the effective imaging time in one line of the CCD 125.

When receiving next horizontal ID data 406, the multiplexer 209 starts constructing a new transmission unit 405, has the horizontal ID data 406 and the white balance coefficient 403 for B as the components of the new transmission unit 405 in that order, and adds image data 407, input from the A/D unit 205 before the inputting of the horizontal ID data 406, as a component of the transmission unit 405 after the last component of the transmission unit 405 (not shown).

As apparent from the above, each transmission unit 405 generated every time the horizontal sync signal 210 is generated is added with the white balance coefficient 402 or 403 alternately and is transmitted in that form to the receiver 4.

The horizontal sync signal 210 which indicates the head of the transmission unit 405 and the TG signal 202 which determines the timing for reading charges from the CCD 125 are generated by the timing generator/sync generator 201 synchronously in such a way that one line of image data 407 of the CCD 125 is sent to the multiplexer 209 at the read timing for the parameter 402 or 403 from the parameter memory 208.

In other words, the multiplexer 209 can detect the timing at which the horizontal ID data 406 is input from the parameter memory 208 as the break of the transmission unit 405, and puts image data which has been input from the A/D unit 205 up to the point of that detection as a component of the transmission unit 405 as one line of image data 407 of the CCD 125.

Fig. 9 is a flowchart of one example of the operations of the capsule endoscope 10 and the receiver 4. When the capsule endoscope 10 is turned ON (YES at step SB1) and starts imaging (step SB2), every time one line of image data 407 of the CCD 125 is read out (YES at step SB3), the one line of image data is multiplexed with one of the R and B white balance coefficients 402 and 403 stored in the parameter memory 208 (step SB4). The multiplexed data is as shown in Fig. 8.

The multiplexed data shown in Fig. 8 is modulated and then transmitted (steps SB5 and SB6). The operation that is performed line by line is carried out similarly for all the lines in one frame of the CCD 125, and is then performed similarly for the next frame (steps SB7 and SB8). These operations are repeated until imaging is stopped (step SB8).

When the receiver 4 receives data sent from the capsule endoscope 10 at step SB6 (YES at step SB11), image data and the white balance coefficient are separated and detected for each one line of image data of the CCD 125 (steps SB12 and SB13). When one line of image data is gathered, white balancing is executed using the white balance coefficient (steps SB14 and SB15). These operations are repeated until the operation of the receiver 4 is finished (step SB16).

Each transmission unit 405 includes the R or B correction coefficient 402 or 403 in the example above. Instead, each transmission unit 405 may consist of plural bits (for example, 8 bits) and contain one bit of the R or B correction coefficient 402 or 403. That is, each transmission unit 405 may be constructed in such a way that the R or B correction coefficient 402 or 403 is identified in plural bits (8 bits in this embodiment) over plural (8 in this embodiment) transmission units 405.

An example in which one transmission unit 405 of data to be transmitted from the capsule endoscope 10 corresponds to one line of image data of the CCD 125 is explained above. Instead of or in addition to the above example, data (frame) 400 which becomes one transmission unit when the data is transmitted from the capsule endoscope 10 can be so constructed as to correspond to one frame of image data of the CCD 125.

As shown in Figs. 10 and 5, when a vertical sync signal (timing data) 210 which is generated by the timing generator/sync generator 201 is input to the parameter memory 208, vertical ID data 401 indicating the beginning of a transmission unit 400 and the parameter 402 or 403 of the white balance coefficient are read into the multiplexer 209 in that order from the parameter memory 208 in response to the input vertical sync signal 210.

When receiving the vertical ID data 401, the multiplexer 209 starts constructing a new transmission unit 400, has the vertical ID data 401, the white balance coefficient 402 for R and the white balance coefficient 403 for B as the components of the new transmission unit 400 in the order they are read from the parameter memory 208, and adds image data 404, output from the A/D unit 205 before the inputting of the vertical ID data 401, as a component of the transmission unit 400 after the last component of the transmission unit 400.

The "image data 404, output from the A/D unit 205 before the inputting of the vertical ID data 401" corresponds to one frame (the pixels of the CCD 125) of data of signal charges accumulated in the vertical shift register (not shown) of the CCD 125 in a vertical retrace line period. In the transmission unit 400, the white balance coefficients 402 and 403 are added to places corresponding to the time before the effective start line of the CCD 125.

The vertical sync signal 210 which indicates the head of the transmission unit 400 and the TG signal 202 which determines the timing for reading charges from the CCD 125 are generated by the timing generator/sync generator 201 synchronously in such a way that the image data 404 constituting one frame of the CCD 125 is sent to the multiplexer 209 from the A/D unit 205 at the timing at which the parameters 402 and 403 are read from the parameter memory 208.

In other words, the multiplexer 209 can detect the timing at which the vertical ID data 401 is input from the parameter memory 208 as the break of the transmission unit 400, and puts image data which has been input from the A/D unit 205 up to the point of that detection as a component of the transmission unit 400 as one frame of image data 404.

As apparent from the above, each transmission unit 400 generated every time the vertical sync signal 210 is generated is added with the white balance coefficients 402 and 403 and is transmitted in that form to the receiver 4.

Data about the white balance coefficients included in each transmission unit 400 is the R and B correction coefficients 402 and 403, whereas data about the white balance coefficients included in each transmission unit 405 is the R or B correction coefficient 402 or 403, or 1-bit data constituting the R or B correction coefficient 402 or 403. The reason why the amount of data about the white balance coefficients included in each transmission unit 405 is smaller than the amount of data about the white balance coefficients included in each transmission unit 400 is because the frequency of occurrence of the horizontal sync signal 210 is higher than the frequency of occurrence of the vertical sync signal 210. That is, even with a smaller amount of data about the white balance coefficients included in each transmission unit 405, each transmission unit 405 is generated at a relatively high frequency, so that the receiver 4 can acquire all the information about the white balance coefficients of the capsule endoscope 10 quickly based on each transmission unit 405.

As shown in Figs. 8 and 10, data is transmitted, with the correction coefficient 402, 403 added thereto, to the receiver 4 for each transmission unit 400, 405. The white balance coefficient of each capsule endoscope 10 is a value which is specifically determined as a value stored in the parameter memory 208 in the fabrication process and does not vary. In this respect, it appears sufficient to send the value to the receiver 4 once, for example, when the capsule endoscope 10 is activated.

The white balance coefficient is however sent to the receiver 4 for each transmission unit 400, 405 in this embodiment to surely avoid the following. With the use of the method of sending the white balance coefficient to the receiver 4 only when the capsule endoscope 10 is activated, if the receiver 4 is not turned ON when the capsule endoscope 10 is activated, for example, the receiver 4 cannot receive the white balance coefficient followed by the display of an image which is not subjected to the white balancing process.

Fig. 11 is a flowchart of the procedures of the white balancing process that is executed by the receiver 4. An example in which the communication from the capsule endoscope 10 to the receiver 4 uses the transmission unit 405 shown in Fig. 8 and the operation according to the flowchart shown in Fig. 9 is performed will be explained.

In the initialization, a detection number i is set equal to 0 in the parameter detector 304 (step SC1). When receiving data of the transmission unit 405 from the demodulator 301, the signal separator 302 of the receiver 4 detects the horizontal ID data 406 from the input data and detects the white balance coefficient 402 or 403 that comes immediately after the horizontal ID data 406. The signal separator 302 separates the horizontal ID data 406 and the white balance coefficient 402 or 403 from the image data 407, sends the image data 407 to the image memory 303, and sends the horizontal ID data 406 and the white balance coefficient 402 or 403 to the parameter detector 304.

The parameter detector 304 acquires the white balance coefficient 402 or 403 immediately following the horizontal ID data 406 and stores the acquired white balance coefficient 402 or 403 in a parameter memory area k(i) in the parameter detector 304 (step SC2). Then, the parameter detector 304 increments the detection number i by 1 (step SC3).

The steps SC2 and SC3 are repeated until the detection number i reaches a preset detection number n (NO at step SC4). The number n corresponds to the number of lines of the CCD 125. When the transmission unit 400 shown in Fig. 10 is used in the communication from the capsule endoscope 10 to the receiver 4; unlike the present example, n corresponds to the number of frames of an image.

As the steps SC2 and SC3 are repeated until the detection number i reaches the detection number n and the white balance coefficient 402 or 403 is stored in n parameter memory areas k(n) in the parameter detector 304, the flow proceeds to step SC5 (YES at step SC4).

As apparent from step SC5, the parameter detector 304 uses data of the white balance coefficient 402 or 403 detected n times, whichever has a high frequency of occurrence, as a white balance coefficient RWB or BWB. This prevents the use of an erroneous white balance coefficient originated from a communication error.

As apparent from step SC6, the image signal processor 305 performs a white balancing process on the image data 407 based on the white balance coefficient RWB or BWB that has been used by the parameter detector 304 at step SC5. With regard to the R pixel, a value Rout obtained by multiplying input data Rin by the white balance coefficient RWB is the result of white balancing process. With regard to the B pixel, a value Bout obtained by multiplying input data Bin by the white balance coefficient BWB is the result of white balancing process.

The first embodiment demonstrates the following advantages.

Since the white balancing process need not be performed by the internal circuits of the capsule endoscope in this embodiment, the circuit scale of the internal circuits does not increase so that the power consumption does not increase. As the white balance coefficient has only to be stored in the parameter memory 208 in this embodiment, the circuit scale of the internal circuits does not increase.

An example of a method in which a chart for white balance is imaged immediately after the capsule endoscope 10 is taken out of the package and is turned ON (before the capsule endoscope 10 is swallowed), an image of the imaged chart is transmitted to the receiver 4, and the receiver 4 acquires the white balance coefficient of the capsule endoscope 10 based on the received image of the chart will be explained. According to the method, when the receiver 4 cannot receive taken image data about the white balance coefficient when the chart is imaged (for example, when the receiver 4 has not been turned ON yet at that time), the image taken by the capsule endoscope 10 does not undergo the white balancing process if the subject 2 has swallowed the capsule endoscope 10 unnoticing the event, and the image taken by the capsule endoscope does not undergo the white balancing process.

According to this embodiment, by way of contrast, even when the receiver 4 cannot receive data sent from the capsule endoscope 10 before the capsule endoscope 10 is swallowed, the capsule endoscope 10 always sends data of the white balance coefficient RWB, BWB together with taken image data to the receiver 4 thereafter. Therefore even when the receiver 4 is turned ON even after the capsule endoscope 10 is swallowed, the taken image can undergo the white balancing process based on the white balance coefficient RWB, BWB received later.

Modifications of the first embodiment will be explained below.

According to the first embodiment, the white balance coefficients RWB and BWB are stored in the parameter memory 208. In a first modification, an R image (Rdata) and a B image (Bdata) with a white chart taken in the fabrication process are stored directly in the parameter memory 208 instead. In this modification, the transmission unit 405, 400 is constructed in such a way that the R image (Rdata) and the B image (Bdata) are included at the place of the white balance coefficient 402 in Fig. 8 or the places of the white balance coefficients 402 and 403 in Fig. 10. The other configuration and operation of the capsule endoscope 10 are the same as those of the first embodiment.

The receiver 4 has a constant Gr to be a reference for R and a constant Gb to be a reference for B, both of which are used in the white balancing process. The receiver 4 receives the R image (Rdata) or the B image (Bdata) and the image data 407 from the received transmission unit 405. The receiver 4 also receives the R image (Rdata) and the B image (Bdata) and the image data 404 from the received transmission unit 400.

In the white balancing process performed on the image data 407, 404 by the receiver 4, a value Rout which is obtained by multiplying data Rin of the image data 407, 404 by (Gr/Rdata) is the result of the white balancing process for the R pixel. Likewise, a value Bout which is obtained by multiplying data Bin of the image data 407, 404 by (Gb/Bdata) is the result of the white balancing process for the B pixel.

The constant Gr to be a reference for R and the constant Gb to be a reference for B can be changed for each location (hospital) where the capsule endoscope 10 is to be used. This can permit the result of the white balancing process to differ depending on the place of usage of the capsule endoscope 10. Even with the same usage place, the constant Gr and the constant Gb can be changed according to the portion of the organ that is imaged by the capsule endoscope 10. Accordingly, the original color of each organ or the color of the pathogenesis to be found in each organ can be reflected in changing the constant Gr and the constant Gb.

With reference to Fig. 12, a second modification of the first embodiment will be explained.

Fig. 12 depicts a modification of the transmission unit 400 in Fig. 10. In the transmission unit 400' in Fig. 12, an error correction code 408 for the R white balance coefficient 402 is added immediately following the R white balance coefficient 402, and an error correction code 409 for the B white balance coefficient 403 is added immediately following the B white balance coefficient 403.

The error correction code 408, 409 is stored together with the white balance coefficient RWB, BWB in the parameter memory 208 when the white balance coefficient RWB, BWB is stored therein in the fabrication process of the capsule endoscope 10. The configuration may be modified in such a way that only the white balance coefficient RWB, BWB is stored in the parameter memory 208 while the error correction code 408, 409 is computed in the capsule endoscope 10 based on the white balance coefficient RWB, BWB read from the parameter memory 208.

The receiver 4 can correct the R white balance coefficient 402 based on the error correction code 408 and can correct the B white balance coefficient 409 based on the error correction code 409.

Though not shown, an error correction code corresponding to the R white balance coefficient 402 can be added between the R white balance coefficient 402 in the transmission unit 405 in Fig. 8 and the image data 407. Likewise, an error correction code corresponding to the B white balance coefficient 403 can be added between the B white balance coefficient 403 and the image data 407.

According to the second modification, in the transmission unit 400, the error correction code 408, 409 is added, together with the white balance coefficient 402, 403, at a place corresponding to a time before the effective start line of the CCD 125. In the transmission unit 405, the error correction code is added, together with the white balance coefficient, at a place corresponding to a time other than the effective imaging time in one line of the CCD 125.

In the second modification, the correct white balance coefficients RWB and BWB can be acquired with a high accuracy even when a communication error occurs. Therefore, the correct white balance coefficients RWB and BWB can be acquired without any problem even when the value of n at step SC4 in Fig. 11 is small.

### (Second Embodiment)

A second embodiment will be explained with reference to Figs. 13 and 14.

According to the second embodiment, pixel defect address data indicating the address of a defective pixel is stored in the parameter memory 208 in addition to the white balance coefficient. Correction of a pixel defect is to correct a defective pixel present at the address of the defective pixel based on the pixel data that corresponds to the addresses around the address of the defective pixel.

The other configuration of the capsule endoscope 10 is the same as that of the first embodiment. The operation of the capsule endoscope 10 and the configuration and operation of the receiver 4 are basically the same as those of the first embodiment.

In the multiplexer 209, image data, the white balance coefficient, and the pixel defect address data are multiplexed and the resultant multiplexed data is sent out from the capsule endoscope 10 via the modulator 211 and the radio unit 142. In the receiver 4, the parameter detector 304 detects the white balance coefficient and the individual parameters of the pixel defect address data, and the image signal processor 305 performs the white balancing process on the image data based on the detected white balance coefficient and performs pixel defect correction based on the detected pixel defect address data. The image that has undergone the white balancing process and pixel defect correction is compressed by the image compressor 306 and the compressed image data is stored in the large-capacity memory 44.

A test is likewise conducted in the fabrication process for each capsule endoscope 10, as done for the white balance coefficient, to acquire the address of each defective pixel of that capsule endoscope 10. The pixel defect address data is written in the parameter memory 208 of each capsule endoscope 10, which is shipped with each pixel defect address data stored in the parameter memory 208 of the capsule endoscope 10.

Fig. 13 is a flowchart of procedures for computing the address of a defective pixel in the fabrication process. First, the CCD 125 is placed at a location where the temperature is set at 50°C (step SD1). This is because a white defect of the CCD 125 is likely to occur at a high temperature. Next, the CCD 125 performs imaging by light-shielding (in a dark room) to find a white defect (step SD2). Then, the address of a pixel of a specified level or more from the base (black) is recorded in the parameter memory 208 as pixel defect address data based on the result of imaging by the CCD 125 at the step SD2 (step SD3). Then, a white chart is imaged by the CCD 125 to find a black defect (step SD4). Next, the address of a pixel of the specified level or less from the base (white) is recorded in the parameter memory 208 as pixel defect address data based on the result of imaging by the CCD 125 at the step SD4 (step SD5).

Next, as shown at step SD6, the pixel defect address data recorded in the parameter memory 208 is verified. The verification is to read pixel defect address data from the parameter memory 208 and check if the read pixel defect address data matches with the address data of the defective pixel detected at step SD3 or SD5.

If the verification result shows no problem (if both addresses are identical), detection of the pixel defect address data is finished.

If the verification result shows some problem, it is determined whether the case with the problem (NG) has occurred a predetermined number of times (step SD7). As the case has not occurred the predetermined number of times (NO at SD7), the flow returns to step SD1.

When the occurrence of the case reaches the predetermined number of times as a result of step SD7 (YES at SD7), the presence of an abnormality in the capsule endoscope 10 (particularly in the parameter memory 208) is displayed (step SD8). The capsule endoscope 10 that has been determined as abnormal will not be shipped as it is.

Fig. 14 depicts transmission data 400' to be a transmission unit when data is transmitted from the capsule endoscope 10 in the second embodiment, and corresponds to Fig. 10 associated with the first embodiment. Like elements explained in the first embodiment are designated by like reference signs and the explanations therefor are omitted.

The transmission unit 400' contains pixel defect address data 410 in addition to the vertical ID data 401, the RWB correction coefficient 402, the BWB correction coefficient 403, and the image data 404.

Though not shown, pixel defect address data can be added between the R white balance coefficient 402 and the image data 407 in the transmission unit 405 in Fig. 8 according to the first embodiment, and pixel defect address data can likewise be added between the B white balance coefficient 403 and the image data 407.

In the second embodiment, in the transmission unit 400, pixel defect address data is added, together with the white balance coefficient 402, 403, at a place corresponding to a time before the effective start line of the CCD 125. In the transmission unit 405, pixel defect address data is added, together with the white balance coefficient, at a place corresponding to a time other than the effective imaging time in one line of the CCD 125.

According to the second embodiment, pixel defect correction of the CCD 125 can be executed.

Either the first modification or the second modification in the first embodiment or both can be adapted to the second embodiment.

Data for correcting a defect originating from a variation in the CCD 125 can be stored in the parameter memory 208. The white balance coefficient and pixel defect address data are one example of such data.

### (Third Embodiment)

A third embodiment will be explained next.

Although the first embodiment explains the example where the CCD 125 is used in the capsule endoscope 10, a CMOS image sensor is used instead of the CCD 125 in the third embodiment. The offset value of the photoelectric conversion characteristic which is specific to each CMOS image sensor is stored in the parameter memory 208 of each capsule endoscope 10 of the third embodiment. The other configuration and operation of the capsule endoscope 10 and the structure and operation of the receiver 4 are basically the same as those of the first embodiment.

In the multiplexer 209, image data and the offset value of the photoelectric conversion characteristic are multiplexed and resultant multiplexed data is sent out from the capsule endoscope 10 via the modulator 211 and the radio unit 142. In the receiver 4, the parameter detector 304 detects the parameter of the offset value of the photoelectric conversion characteristic, and the image signal processor 305 corrects the photoelectric conversion characteristic with respect to the image data based on the detected offset value of the photoelectric conversion characteristic. The image whose photoelectric conversion characteristic has been corrected is compressed by the image compressor 306 and the compressed image data is stored in the large-capacity memory 44.

A test is conducted in the fabrication process for each capsule endoscope 10, as done for the white balance coefficient in the first embodiment, to acquire the offset value of the photoelectric conversion characteristic of that capsule endoscope 10. The offset value of the photoelectric conversion characteristic is written in the parameter memory 208 of each capsule endoscope 10, which is shipped with the offset value of each photoelectric conversion characteristic stored in the parameter memory 208 of the capsule endoscope 10.

Fig. 15 is a graph for explaining a way of acquiring the offset value of the photoelectric conversion characteristic of each imaging device (for example, a CMOS image sensor). As shown in Fig. 15, signal outputs when lights of different luminous energies are input to each imaging device are obtained and plotted as points A and B. The points A and B are connected by a line whose intersection with the Y axis is acquired as the offset value of the photoelectric conversion characteristic of the imaging device.

According to the third embodiment, it is possible to correct the photoelectric conversion characteristic when an imaging device is used as the solid-state imaging device of the capsule endoscope 10.

Although added information such as the white balance coefficient 402, 403, the error correction code 408, 409, the pixel defect address data 410, or the offset value of the photoelectric conversion characteristic is added in front of the image data 404 before being sent out in any one of the first to the third embodiments, it is preferable to add the added information on the rear end side of the image data 404, and, it is more preferable to add the added information at the rear end of the image data 404.

Fig. 16 depicts a configuration where the white balance coefficients 402 and 403 are added at the rear end of the image data 404. When such added information is added at the rear end of the image data 404, the receiver can receive data with synchronization established by the vertical sync signal more reliably. When the frame 400 is sent and received discretely, particularly, a resynchronization process should be performed each time, so that it is preferable to place added information at the place where stable synchronization is taken. While added information consists of two bytes at the most in the example of Fig. 16, for example, the added information, which significantly affects the restoration of image data, should preferably be added at the rear end of the image data 404. With this, the receiver can acquire stable and reliable added information.

### (Fourth Embodiment)

A fourth embodiment will be explained next.

In the first embodiment, digital transmission is performed, whereas it is analog transmission in the fourth embodiment. Like elements explained in the first embodiment are designated by like reference signs and the explanations therefor are omitted.

As shown in Fig. 17, an image processor 143a' of the capsule endoscope 10 sends analog image data, output from the CCD 125, as an analog signal to the modulator 211. Because of analog transmission, there is no A/D converter 205 as shown in Fig. 5. The white balance coefficients RWB and BWB are stored in the parameter memory 208 as in the parameter memory 208 of the first embodiment.

As shown in Fig. 17, a multiplexer 209' of the image processor 143a' has a mixer 212 and an adder 213. In response to the timing signal 210, the white balance coefficient RWB, BWB is read from the parameter memory 208 and sent to the mixer 212 where the white balance coefficient RWB, BWB is mixed with a sync signal SG1. The adder 213 superimposes the mixing result from the mixer 212 and image data. The output of the adder 213 is frequency-modulated by the modulator 211.

For analog transmission, as apparent from the above, the sync signal SG1 output from the timing generator/sync generator 201 is superimposed directly with the image data by the multiplexer 209' to thereby identify the break between images from a plurality of images contained in the image data.

Fig. 19 depicts an output signal S1 from the multiplexer 209' in Fig. 17. As shown in Fig. 19, in analog transmission, signals are transmitted in the form of a signal waveform similar to that of an NTSC composite video signal. In Fig. 19, a portion 601 above a reference level 600 is a video signal (corresponding to image data) and a portion below the level is the sync signal SG 1. A reference sign 602 is a horizontal sync signal. The white balance coefficients RWB and BWB are mixed with the sync signal SG1 below the reference level 600 by the mixer 212. A reference sign 603 is a vertical sync signal.

As shown in Figs. 19 and 17, the vertical sync signal 603 and the horizontal sync signal 602 (sync signal SG1) are mixed with the white balance coefficients RWB and BWB in the mixer 212, and the mixing result is mixed with the video signal 601 in the adder 213. As shown in Fig. 19, the white balance coefficients RWB and BWB are superimposed at the back of the vertical sync signal 603 and are added at a place corresponding to the time before the effective start line of the CCD 125 (to the left from the video signal 601).

As shown in Fig. 19, the vertical sync signal 603 which is set to a low level over a long period of time is detected as it is put through an LPF (Low-Pass Filter) in the receiver 4. The horizontal sync signal 602 is detected as it is put through a BPF (Band-Pass Filter) in the receiver 4. As it is predetermined that the white balance coefficients RWB and BWB are present after a predetermined clock from the detection of the horizontal sync signal 602, the white balance coefficients RWB and BWB can be detected easily (see Fig. 18 to be discussed later).

Fig. 20 is another example of the output signal S1 from the multiplexer 209' in Fig. 17. In Fig. 20, as in Fig. 19, the white balance coefficients RWB and BWB are mixed with the sync signal SG1 (portion below the reference level 600) and are superimposed on the vertical sync signal 603. However, Fig. 20 differs from Fig. 19 in that the location where mixing takes place comes after the video signal 601 (the location is in front of the video signal 601 in Fig. 19).

In Fig. 20, coefficient ID signals 605a and 605b indicating the presence of the white balance coefficients RWB and BWB are added immediately before the respective white balance coefficients RWB and BWB. As the receiver 4 detects the coefficient ID signals 605a and 605b, it is possible to identify the presence of the white balance coefficients RWB and BWB immediately after the coefficient ID signals 605a and 605b. When both of the R and B white balance coefficients RWB and BWB are laid out consecutively, the coefficient ID signal 605a alone is sufficient, and the coefficient ID signal 605b is unnecessary. The coefficient ID signals 605a and 605b can be added immediately before the respective white balance coefficients RWB and BWB also in the example of Fig. 19.

Figs. 19 and 20 are examples where both the R and B white balance coefficients RWB and BWB are superimposed on each vertical sync signal 603. Figs. 21 and 22 are examples where only 1-bit data of the white balance coefficient RWB or BWB (consisting of eight bits D7 to D0) are superimposed on each horizontal sync signal 602. The 1-bit data of the white balance coefficient RWB or BWB is added at a place corresponding to a time other than the effective imaging time in one line of the CCD 125.

The reason for the amount of data about the white balance coefficients which is to be superimposed on the horizontal sync signal 602 being smaller than the amount of data about the white balance coefficients to be superimposed on the vertical sync signal 603 is because the frequency of occurrence of the horizontal sync signal 602 is higher than the frequency of occurrence of the vertical sync signal 603 as mentioned above.

In Fig. 21 (a), as 1-bit white balance coefficients (D7-D0) respectively superimposed on eight horizontal sync signals 602 are arranged in order, the R white balance coefficient RWB is detected, and as 1-bit white balance coefficients (D7-D0) respectively superimposed on next eight horizontal sync signals 602 are arranged in order, the B white balance coefficient RWB is detected.

Fig. 21 (b) is an example where the timing of superimposing the horizontal sync signal 602 is shifted. In Fig. 21(b), unlike in Fig. 21 (a), data is inserted immediately before the falling of the horizontal sync signal. This structure makes the detection of the white balance coefficient easier when the horizontal sync signal is detected at the rising edge. Since the width of the horizontal sync signal becomes narrower if the white balance coefficient is at a high level (H), it is possible to detect whether the inserted coefficient is at H or L based on the level duration of the horizontal sync signal.

Fig. 22, unlike Fig. 21, depicts that the same 1-bit data of the white balance coefficient RWB or BWB is superimposed on the consecutive three horizontal sync signals 602. The receiver 4 detects the 1-bit data of the white balance coefficient RWB or BWB superimposed every three horizontal sync signals 602.

When the white balance coefficient to be superimposed on one horizontal sync signal 602 can not be read out in the receiver 4, the accurate white balance coefficient RWB or BWB cannot be acquired. In Fig. 22, by way of contrast, even if the one bit superimposed on, for example, the second horizontal sync signal 602 is erroneously identified as the one bit superimposed on the first horizontal sync signal 602, it can be identified correctly as D7 and the one bit superimposed on the third horizontal sync signal 602 from D7 can be identified correctly as D6. In Fig. 22, in setting D7, three line coefficients from the first sync signal are referred to settle data with a high frequency of occurrence as the white balance coefficient.

As shown in Fig. 18, an image processor 300' of the receiver 4, unlike the image processor 300 at the time of digital transmission shown in Fig. 6, is added with an A/D converter 307. A signal separator 302' of the image processor 300' has a clamp circuit 701, a sync-signal separator 702, a vertical-sync detector 703, a horizontal-sync detector 704 and a line-number detector 705.

The clamp circuit 701 clamps an output signal from the demodulator 301 and detects the reference level 600 to separate the sync signal (horizontal sync signal 602 and vertical sync signal 603) SG1 and the video signal 601.

The sync-signal separator 702 separates the sync signal SG 1 and outputs the video signal 601 to the A/D converter 307. The sync signal SG1 is sent to the vertical-sync detector 703 and the horizontal-sync detector 704. The vertical-sync detector 703 detects the vertical sync signal 603, while the horizontal-sync detector 704 detects the horizontal sync signal 602. The detection result from each of the vertical-sync detector 703 and the horizontal-sync detector 704 is sent to the line-number detector 705.

It is known beforehand in the line-number detector 705 that the R white balance coefficient RWB is included at a point a predetermined clock after the horizontal sync signal 602 in the second line from the vertical sync signal 603 and the B white balance coefficient BWB is included at a point a predetermined clock after the horizontal sync signal 602 in the third line in the example in Fig. 19, for example.

The line-number detector 705 sends the parameter detector 304 a sampling phase output instructing a point a predetermined clock after the horizontal sync signal 602 in the second line from the vertical sync signal 603 and a point a predetermined clock after the horizontal sync signal 602 in the third line. The parameter detector 304 can acquire the white balance coefficients RWB and BWB from the sync signal SG1 based on the sampling phase output.

Modifications of the fourth embodiment will be explained with reference to Figs. 23 and 24.

Fig. 23 depicts a modification of the image processor in Fig. 17. A multiplexer 209" has a mixer 212', an adder 213' and a D/A converter 214. The white balance coefficients RWB and BWB read from the parameter memory 208 are converted to analog signals in the D/A converter 214, and are then mixed with image data in the mixer 212'. The adder 213' superimposes the mixing result from the mixer 212' and the sync signal SG 1. The output of the adder 213' is frequency-modulated by the modulator 211.

Fig. 24 depicts an output signal S2 from the multiplexer 209". As shown in Fig. 24, the white balance coefficients RWB and BWB are mixed with the image data 601 above the reference level 600 in the mixer 212'. The white balance coefficient RWB is superimposed on the image data 601 in the second line after the first horizontal sync signal 602 after the vertical sync signal 603 has risen, and the white balance coefficient BWB is superimposed on the image data 601 in the third line after the second horizontal sync signal 602. The actual video signal 601 starts at the fourth line after the third horizontal sync signal 602.

Although the white balance coefficients RWB and BWB are added in front of a series of video signals 601 or in a dispersed manner before being sent out in the fourth embodiment, it is preferable that the white balance coefficients RWB and BWB are added on the rear end of a series of video signals 601. It is more preferable that the white balance coefficients RWB and BWB are added at the rear end of a series of video signals 601.

Fig. 25 depicts a structure where the white balance coefficients RWB and BWB are added at the rear end of a series of n video signals 601. With the white balance coefficients RWB and BWB added at the rear end of a series of video signals 601, the receiver can receive data with synchronization more surely taken by the vertical sync signal 603. While added information such as the white balance coefficient RWB, BWB consists of two bytes at the most in the example of Fig. 16, for example, the added information, which significantly affects the restoration of image data, should preferably be added at the rear end of a series of video signals 601. In this instance, the receiver can acquire stable and reliable added information. It is also preferable that added information, such as the error correction codes 408 and 409, the pixel defect address data 410, and the offset value of the photoelectric conversion characteristic, other than the white balance coefficients RWB and BWB, are added at the rear end of a series of video signals 601.

### (Fifth Embodiment)

A fifth embodiment will be explained with reference to Figs. 26 and 27.

In the fifth embodiment, like elements explained in the first embodiment are designated by like reference signs and the explanations therefor are omitted. An example in which the capsule endoscope 10 performs analog transmission will be explained.

In the fifth embodiment, unlike the first embodiment, the white balance coefficient stored in the parameter memory 208 is modulated alone and transmitted without being multiplexed with an image signal, or an image signal is modulated alone and transmitted. The receiver 4 demodulates two modulated signals to acquire the white balance coefficient and an image signal.

As shown in Fig. 26, the image processor 143a of the capsule endoscope 10, unlike the one in Fig. 3, does not have the multiplexer 209 because the white balance coefficient is not multiplexed with an image signal in the fifth embodiment. A signal processing/control unit 143' shown in Fig. 26 has two modulators 211a and 211b.

The modulator 211a modulates the white balance coefficient stored in the parameter memory 208 at a carrier frequency f1. The modulator 211 b modulates an image signal at a carrier frequency f2. The transmitting unit 142a amplifies the modulated signal of the white balance coefficient output from the modulator 211a and amplifies the modulated signal of the image signal output from the modulator 211 b. The common antenna 142b transmits the modulated signals of different carrier frequencies f1 and f2, amplified by the transmitting unit 142a.

As shown in Fig. 27, the receiver 4, unlike the one in Fig. 4, has two demodulators 301 a and 301 b and has the parameter detector 304 provided outside the image processor 300. Signals of radio waves (the modulated signal of the white balance coefficient and the modulated signal of the image signal) caught by the common antennas 31 to 34 are amplified by the receiving unit 41.

The demodulator 301a demodulates the modulated signal of the carrier frequency f1 and sends the demodulated signal to the parameter detector 304. The parameter detector 304 detects the white balance coefficient based on the input signal.

The demodulator 301 b demodulates the modulated signal of the carrier frequency f2 and sends the demodulated signal to the image processor 300. The signal separator 302 in the image processor 300 separates an image signal and a sync signal. By using the sync signal, the image processor 300 accesses the parameter detector 304 to acquire the white balance coefficient from the parameter detector 304. The image processor 300 performs the white balancing process on the image signal using the white balance coefficient.

Although an example of analog transmission is explained above, the fifth embodiment is feasible for digital transmission. In this instance, the operation of the capsule endoscope 10 and the operations of the components of the receiver 4 up to the demodulators 301a and 301 b are the same in digital transmission. Since the image processor 300 of the receiver 4 in digital transmission need not separate an image signal and a sync signal, the signal separator 302 is unnecessary and the white balancing process should be performed on the image signal by using the white balance coefficient detected by the parameter detector 304.

The method of transmitting the white balance coefficient stored in the parameter memory 208 and an image signal separately without being multiplexed and demodulating the white balance coefficient and the image signal separately in the receiver 4 as done in the fifth embodiment can bring about advantages similar to those of the first embodiment.

The capsule endoscope according to the present invention has low power consumption in signal processing which is specific to an imaging device.

### INDUSTRIAL APPLICABILITY

As described above, the present invention relates to a medical endoscope, and is particularly suitable for a swallowable type capsule endoscope which takes an internal image of a celom, and a capsule endoscope system which uses the capsule endoscope.

## Claims

1. A capsule endoscope comprising:
a storage unit that stores signal processing data necessary for signal processing specific to an imaging device of the capsule endoscope; and
a transmitting unit that transmits the signal processing data stored in the storage unit.

2. The capsule endoscope according to claim 1, wherein the signal processing data is a value acquired before shipment of the capsule endoscope in advance.

3. The capsule endoscope according to claim 1, wherein the signal processing data is data of a white balance coefficient to be used when a white balancing process of the imaging device is performed.

4. The capsule endoscope according to claim 1, wherein the signal processing data is data of an image of a chart for color signal processing which is taken by the imaging device.

5. The capsule endoscope according to claim 1, wherein the signal processing data is data indicating an address of a defective pixel of the imaging device.

6. The capsule endoscope according to claim 1, wherein the signal processing data is data indicating an offset value of the photoelectric conversion characteristic of the imaging device.

7. The capsule endoscope according to claim 1, wherein the transmitting unit transmits the signal processing data together with imaged data taken by the imaging device.

8. The capsule endoscope according to claim 7, wherein the transmitting unit transmits the imaged data with at least a part of the signal processing data included in each frame to be a transmission unit at a time of transmitting the imaged data.

9. The capsule endoscope according to claim 8, wherein the signal processing data is added on an end side of the frame.

10. The capsule endoscope according to claim 8, wherein the signal processing data is added to a top end of the frame.

11. The capsule endoscope according to claim 1, wherein the transmitting unit transmits the signal processing data together with an error correction code of the signal processing data.

12. The capsule endoscope according to claim 11, wherein the error correction code is acquired before shipment of the capsule endoscope in advance, and data of the error correction code is stored in the storage unit.

13. A capsule endoscope system comprising:
a capsule endoscope including
a storage unit that stores signal processing data necessary for signal processing specific to an imaging device of the capsule endoscope; and
a transmitting unit that transmits the signal processing data stored in the storage unit; and
a receiver that receives the signal processing data transmitted from the transmitting unit, wherein
the capsule endoscope does not perform signal processing specific to the imaging device but the receiver performs signal processing specific to the imaging device based on the received signal processing data.

14. The capsule endoscope system according to claim 13, wherein the signal processing data is a value acquired before shipment of the capsule endoscope in advance.

15. The capsule endoscope according to claim 13, wherein the signal processing data is data of a white balance coefficient to be used when a white balancing process of the imaging device is performed.

16. The capsule endoscope system according to claim 13, wherein the signal processing data is data of an image of a chart for color signal processing which is taken by the imaging device.

17. The capsule endoscope system according to claim 13, wherein the signal processing data is data indicating an address of a defective pixel of the imaging device.

18. The capsule endoscope system according to claim 13, wherein the signal processing data is data indicating an offset value of the photoelectric conversion characteristic of the imaging device.

19. The capsule endoscope system according to claim 13, wherein the transmitting unit transmits the signal processing data together with imaged data taken by the imaging device.

20. The capsule endoscope system according to claim 19, wherein the transmitting unit transmits the imaged data with at least a part of the signal processing data included in each frame to be a transmission unit at a time of transmitting the imaged data.

21. The capsule endoscope system according to claim 19, wherein the signal processing data is added on an end side of the frame.

22. The capsule endoscope system according to claim 19, wherein the signal processing data is added to a top end of the frame.

23. The capsule endoscope according to claim 13, wherein the transmitting unit transmits the signal processing data together with an error correction code of the signal processing data.

24. The capsule endoscope system according to claim 23, wherein the error correction code is acquired before shipment of the capsule endoscope in advance, and data of the error correction code is stored in the storage unit.
